(19) Europäisches Patentamt / European Patent Office / Office européen des brevets

(11) **EP 4 782 464 A1**

(12) **EUROPEAN PATENT APPLICATION**
published in accordance with Art. 153(4) EPC

(43) Date of publication:
29.07.2026 Bulletin 2026/31

(21) Application number: 24867554.8

(22) Date of filing: 20.09.2024

(51) International Patent Classification (IPC):
C07K 16/40 (2006.01)        C07K 19/00 (2006.01)
C12N 15/13 (2006.01)        C12N 15/62 (2006.01)
C12N 15/85 (2006.01)        C12N 5/078 (2010.01)
A61K 39/395 (2006.01)       A61P 35/00 (2006.01)

(52) Cooperative Patent Classification (CPC):
A61K 39/395; A61P 35/00; C07K 16/00;
C07K 16/40; C07K 19/00; C12N 5/06; C12N 15/62;
C12N 15/85

(86) International application number:
PCT/CN2024/120038

(87) International publication number:
WO 2025/061149 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.09.2023 PCT/CN2023/120440

(71) Applicants:
• Nanjing Legend Biotech Co., Ltd.
Nanjing, Jiangsu 211100 (CN)
• Legend Biotech Ireland Limited
Dublin 18, D18 T934 (IE)

(72) Inventors:
• XU, Panglian
Nanjing, Jiangsu 211100 (CN)
• ZHUANG, Qiuchuan
Nanjing, Jiangsu 211100 (CN)
• WEI, Min
Nanjing, Jiangsu 211100 (CN)
• DING, Kaiqiang
Nanjing, Jiangsu 211100 (CN)

(74) Representative: Murgitroyd & Company
165-169 Scotland Street
Glasgow G5 8PL (GB)

Remarks:
The complete document including Reference
Table(s) and the Sequence Listing(s) can be
downloaded from the EPO website

(54) **ANTI-GCC ANTIBODY, CHIMERIC ANTIGEN RECEPTOR, AND USE**

(57) The present disclosure relates to the field of biological medicines, and in particular to an anti-guanylyl cyclase C (GCC) antibody or an antigen binding fragment, a chimeric antigen receptor (CAR) containing the anti-GCC antibody or the antigen binding fragment, and a use. The disclosed anti-GCC antibody or antigen-binding fragment can be used for companion diagnostics of GCC-related diseases or conditions, e.g., immunohistochemical analysis. The disclosed GCC CAR construct-transduced immune cells can be used for cellular immunotherapy of GCC-related diseases or conditions.

*FIG. 3*

**Description**

**Cross-Reference to Related Applications**

The present application is based on and claims the right of priority for the PCT application with the application number of PCT/CN 2023/120440 and the filling date of September 21, 2023, the entire content of which is hereby incorporated by reference into the present application in its entirety.

**Technical Field**

The present disclosure belongs to the field of biological medicines, and relates to an anti-GCC antibody, a chimeric antigen receptor containing the anti-GCC antibody, and a use.

**Background Art**

Guanylyl cyclase C (GUCY2C or GCC) is a membrane-bound guanylyl cyclase that is a single-pass transmembrane protein receptor, comprising an extracellular domain, a transmembrane domain, and an intracellular domain. This enzyme is an intestinal receptor for bacterial heat-stable enterotoxin, so GUCY2C is also known as a heat-stable enterotoxin receptor. Guanylin and uroguanylin, as well as synthetic GCC agonists, can bind to and activate GCC. Activation of GCC stimulates the production of the intracellular second messenger cGMP, which functions to regulate water and electrolyte balance, maintain intestinal barrier function, and exert an antiinflammatory activity.

In normal human tissues, GCC is expressed at the apical surface of intestinal epithelial cells. Loss-of-function mutations in GCC result in decreased fluid and ion secretion, leading to constipation; gain-of-function mutations in GCC increase fluid and ion secretion, resulting in secretory diarrhoea. The GCC-cGMP signalling axis also plays an important role in the pathogenesis of diseases such as intestinal tumours and inflammatory bowel diseases: Inactivation of GCC-cGMP signalling due to significant down-regulation of guanylin and uroguanylin is closely associated with tumourigenesis. Up-regulation of GCC-cGMP signalling activates the STAT1 signalling pathway, leading to inflammatory bowel diseases, etc. GCC is specifically and highly expressed in most gastrointestinal tumour tissues and is therefore an ideal target for targeted treatment of gastrointestinal tumours.

Chimeric antigen receptor T (CAR-T) cell immunotherapy is a novel antigen-targeted precision therapy technology. After transfection and expression of specific CAR genes, T cells can recognise and bind to specific antigens on the surface of tumour cells, activating the T cells independently of MHC co-stimulatory signals, thereby attacking the tumour cells. CAR-T cell therapy has potential advantages over other T cell redirection therapeutic strategies. Other therapeutic strategies often require long-term therapy until disease progression, whereas CAR-T cells can expand in vivo, achieving long-term disease remission with only a single infusion. CAR-T cell therapy is emerging as a promising therapeutic regimen in cancer treatment.

Currently, there is still a need to develop novel anti-GCC antibodies.

**Summary**

After in-depth research and creative work, the present disclosure provides a novel anti-guanylyl cyclase C (GCC) antibody, which can recognise human and mouse GCC antigen targets, exhibits strong cell membrane localisation and high specificity, and can be used for the development of companion diagnostic antibodies in the future. The rabbit-derived antibody targeting GCC provided in the present application can not only be used to develop a chimeric antigen receptor (CAR) targeting GCC but also be used for immunohistochemical analysis. The following invention is thus provided:
One aspect of the present disclosure relates to an anti-GCC antibody or an antigen-binding fragment thereof, comprising a heavy chain variable region and a light chain variable region, the heavy chain variable region comprising heavy chain complementarity determining regions HCDR1 to HCDR3, and the light chain variable region comprising light chain complementarity determining regions LCDR1 to LCDR3, wherein

(1) the heavy chain variable region comprises HCDR1, HCDR2, and HCDR3 in an amino acid sequence set forth in SEQ ID NO: 31, and the light chain variable region comprises LCDR1, LCDR2, and LCDR3 in an amino acid sequence set forth in SEQ ID NO: 37;

(2) the heavy chain variable region comprises HCDR1, HCDR2, and HCDR3 in an amino acid sequence set forth in SEQ ID NO: 32, and the light chain variable region comprises LCDR1, LCDR2, and LCDR3 in an amino acid sequence set forth in SEQ ID NO: 38;

(3) the heavy chain variable region comprises HCDR1, HCDR2, and HCDR3 in an amino acid sequence set forth in SEQ ID NO: 33, and the light chain variable region comprises LCDR1, LCDR2, and LCDR3 in an amino acid sequence set forth in SEQ ID NO: 39;

(4) the heavy chain variable region comprises HCDR1, HCDR2, and HCDR3 in an amino acid sequence set forth in SEQ ID NO: 34, and the light chain variable region comprises LCDR1, LCDR2, and LCDR3 in an amino acid sequence set forth in SEQ ID NO: 40; or

(5) the heavy chain variable region comprises HCDR1, HCDR2, and HCDR3 in an amino acid sequence set forth in SEQ ID NO: 35, and the light chain variable region comprises LCDR1, LCDR2, and LCDR3 in an amino acid sequence set forth in SEQ ID NO: 41.

In some embodiments of the present disclosure, in the anti-GCC antibody or the antigen-binding fragment thereof,
the HCDR1 to HCDR3 of the heavy chain variable region and the LCDR1 to LCDR3 of the light chain variable region are determined according to the Kabat numbering system, the AbM numbering system, the IMGT numbering system, the Chothia numbering system or the Contact numbering system.

In some embodiments of the present disclosure, in the anti-GCC antibody or the antigen-binding fragment thereof,

(1) the amino acid sequence of the HCDR1 is set forth in SEQ ID NO: 1, the amino acid sequence of the HCDR2 is set forth in SEQ ID NO: 6, the amino acid sequence of the HCDR3 is set forth in SEQ ID NO: 11, the amino acid sequence of the LCDR1 is set forth in SEQ ID NO: 16, the amino acid sequence of the LCDR2 is set forth in SEQ ID NO: 21, and the amino acid sequence of the LCDR3 is set forth in SEQ ID NO: 26;

(2) the amino acid sequence of the HCDR1 is set forth in SEQ ID NO: 2, the amino acid sequence of the HCDR2 is set forth in SEQ ID NO: 7, the amino acid sequence of the HCDR3 is set forth in SEQ ID NO: 12, the amino acid sequence of the LCDR1 is set forth in SEQ ID NO: 17, the amino acid sequence of the LCDR2 is set forth in SEQ ID NO: 22, and the amino acid sequence of the LCDR3 is set forth in SEQ ID NO: 27;

(3) the amino acid sequence of the HCDR1 is set forth in SEQ ID NO: 3, the amino acid sequence of the HCDR2 is set forth in SEQ ID NO: 8, the amino acid sequence of the HCDR3 is set forth in SEQ ID NO: 13, the amino acid sequence of the LCDR1 is set forth in SEQ ID NO: 18, the amino acid sequence of the LCDR2 is set forth in SEQ ID NO: 23, and the amino acid sequence of the LCDR3 is set forth in SEQ ID NO: 28;

(4) the amino acid sequence of the HCDR1 is set forth in SEQ ID NO: 4, the amino acid sequence of the HCDR2 is set forth in SEQ ID NO: 9, the amino acid sequence of the HCDR3 is set forth in SEQ ID NO: 14, the amino acid sequence of the LCDR1 is set forth in SEQ ID NO: 19, the amino acid sequence of the LCDR2 is set forth in SEQ ID NO: 24, and the amino acid sequence of the LCDR3 is set forth in SEQ ID NO: 29; or

(5) the amino acid sequence of the HCDR1 is set forth in SEQ ID NO: 5, the amino acid sequence of the HCDR2 is set forth in SEQ ID NO: 10, the amino acid sequence of the HCDR3 is set forth in SEQ ID NO: 15, the amino acid sequence of the LCDR1 is set forth in SEQ ID NO: 20, the amino acid sequence of the LCDR2 is set forth in SEQ ID NO: 25, and the amino acid sequence of the LCDR3 is set forth in SEQ ID NO: 30.

In some embodiments of the present disclosure, in the anti-GCC antibody or the antigen-binding fragment thereof,

(1) the heavy chain variable region comprises an amino acid sequence having at least 80%, at least 85%, at least 90%, at least 95%, at least 96%, at least 97%, at least 98% or at least 99% identity to the amino acid sequence set forth in SEQ ID NO: 31, and the light chain variable region comprises an amino acid sequence having at least 80%, at least 85%, at least 90%, at least 95%, at least 96%, at least 97%, at least 98% or at least 99% identity to the amino acid sequence set forth in SEQ ID NO: 37;

(2) the heavy chain variable region comprises an amino acid sequence having at least 80%, at least 85%, at least 90%, at least 95%, at least 96%, at least 97%, at least 98% or at least 99% identity to the amino acid sequence set forth in SEQ ID NO: 32, and the light chain variable region comprises an amino acid sequence having at least 80%, at least 85%, at least 90%, at least 95%, at least 96%, at least 97%, at least 98% or at least 99% identity to the amino acid sequence set forth in SEQ ID NO: 38;

(3) the heavy chain variable region comprises an amino acid sequence having at least 80%, at least 85%, at least 90%, at least 95%, at least 96%, at least 97%, at least 98% or at least 99% identity to the amino acid sequence set forth in SEQ ID NO: 33, and the light chain variable region comprises an amino acid sequence having at least 80%, at least 85%, at least 90%, at least 95%, at least 96%, at least 97%, at least 98% or at least 99% identity to the amino acid sequence set forth in SEQ ID NO: 39;

(4) the heavy chain variable region comprises an amino acid sequence having at least 80%, at least 85%, at least 90%, at least 95%, at least 96%, at least 97%, at least 98% or at least 99% identity to the amino acid sequence set forth in SEQ ID NO: 34, and the light chain variable region comprises an amino acid sequence having at least 80%, at least 85%, at least 90%, at least 95%, at least 96%, at least 97%, at least 98% or at least 99% identity to the amino acid sequence set forth in SEQ ID NO: 40; or

(5) the heavy chain variable region comprises an amino acid sequence having at least 80%, at least 85%, at least 90%, at least 95%, at least 96%, at least 97%, at least 98% or at least 99% identity to the amino acid sequence set forth in SEQ ID NO: 35, and the light chain variable region comprises an amino acid sequence having at least 80%, at least 85%, at least 90%, at least 95%, at least 96%, at least 97%, at least 98% or at least 99% identity to the amino acid sequence set forth in SEQ ID NO: 41.

In some embodiments of the present disclosure, in the anti-GCC antibody or the antigen-binding fragment thereof,

(1) the amino acid sequence of the heavy chain variable region is set forth in SEQ ID NO: 31, and the amino acid sequence of the light chain variable region is set forth in SEQ ID NO: 37;
(2) the amino acid sequence of the heavy chain variable region is set forth in SEQ ID NO: 32, and the amino acid sequence of the light chain variable region is set forth in SEQ ID NO: 38;
(3) the amino acid sequence of the heavy chain variable region is set forth in SEQ ID NO: 33, and the amino acid sequence of the light chain variable region is set forth in SEQ ID NO: 39;
(4) the amino acid sequence of the heavy chain variable region is set forth in SEQ ID NO: 34, and the amino acid sequence of the light chain variable region is set forth in SEQ ID NO: 40; or
(5) the amino acid sequence of the heavy chain variable region is set forth in SEQ ID NO: 35, and the amino acid sequence of the light chain variable region is set forth in SEQ ID NO: 41.

In some embodiments of the present disclosure, in the anti-GCC antibody or the antigen-binding fragment thereof, the anti-GCC antibody comprises a non-CDR region, and the non-CDR region is from a human, a mouse, or a rabbit; in some specific embodiments, the non-CDR region comprises a framework region, a heavy chain constant region, and/or a light chain constant region.

In some embodiments of the present disclosure, in the anti-GCC antibody or the antigen-binding fragment thereof, the antigen-binding fragment is a Fab, a Fab', a F(ab')2, a single chain variable fragment (scFv), or a disulphide-stabilised variable fragment (dsFv).

In some embodiments of the present disclosure, the anti-GCC antibody or the antigen-binding fragment thereof comprises an amino acid sequence having at least 80%, at least 85%, at least 90%, at least 95%, at least 96%, at least 97%, at least 98%, or at least 99% identity to any one of the sequences of SEQ ID NO: 43 to SEQ ID NO: 47, or comprises an amino acid sequence having at least 80%, at least 85%, at least 90%, at least 95%, at least 96%, at least 97%, at least 98% or at least 99% identity to SEQ ID NO: 57 and SEQ ID NO: 58; in some specific embodiments, the amino acid sequence of the anti-GCC antibody or the antigen-binding fragment thereof is set forth in any one of the sequences of SEQ ID NO: 43 to SEQ ID NO: 47.

Another aspect of the present disclosure relates to an isolated nucleic acid, which encodes the anti-GCC antibody or the antigen-binding fragment thereof according to any one in the present disclosure.

A further aspect of the present disclosure relates to a vector, which comprises the isolated nucleic acid of the present disclosure.

A further aspect of the present disclosure relates to a host cell, which comprises the isolated nucleic acid of the present disclosure, or comprises the vector of the present disclosure.

A further aspect of the present disclosure relates to a chimeric antigen receptor (CAR), which comprises a polypeptide, wherein the polypeptide comprises:

(1) an extracellular antigen-binding domain, wherein the extracellular antigen-binding domain comprises the anti-GCC antibody or the antigen-binding fragment according to any one in the present disclosure;
(2) a transmembrane domain; and
(3) an intracellular signalling domain.

In some embodiments of the present disclosure, in the chimeric antigen receptor, the anti-GCC antibody or the antigen-binding fragment is a single chain variable fragment (scFv).

In some embodiments of the present disclosure, the chimeric antigen receptor further comprises a signal peptide located at the N-terminus of the polypeptide;

in some specific embodiments, the signal peptide is derived from a CD8α molecule, a CD28 molecule, or a CD4 molecule;
in some specific embodiments, the signal peptide comprises an amino acid sequence set forth in SEQ ID NO: 61, or an amino acid sequence having at least 80%, at least 85%, at least 90%, at least 95%, at least 96%, at least 97%, at least 98% or at least 99% identity to SEQ ID NO: 61.

In some embodiments of the present disclosure, the chimeric antigen receptor further comprises a hinge domain located between the C-terminus of the extracellular antigen-binding domain and the N-terminus of the transmembrane domain;

in some specific embodiments, the hinge region is derived from IgG4Fc CH2CH3, CD28, or CD8$\alpha$;
in some specific embodiments, the hinge region comprises an amino acid sequence set forth in SEQ ID NO: 62, or an amino acid sequence having at least 80%, at least 85%, at least 90%, at least 95%, at least 96%, at least 97%, at least 98% or at least 99% identity to SEQ ID NO: 62.

In some embodiments of the present disclosure, in the chimeric antigen receptor, the transmembrane domain is derived from one or more of the transmembrane regions of CD8$\alpha$, CD4, CD28, CD137, CD80, CD86, CD152, and PD1 molecules;
in some specific embodiments, the transmembrane domain comprises an amino acid sequence set forth in SEQ ID NO: 63, or an amino acid sequence having at least 80%, at least 85%, at least 90%, at least 95%, at least 96%, at least 97%, at least 98% or at least 99% identity to SEQ ID NO: 63.

In some embodiments of the present disclosure, in the chimeric antigen receptor, the intracellular signalling domain comprises a costimulatory signalling domain;

in some specific embodiments, the costimulatory signalling domain is derived from the following costimulatory molecules: CD27, CD28, CD137, OX40, CD30, CD40, CD3, HVEM, ICOS, Myd88, LFA-1, ICOS, CD2, CD7, LIGHT, NKG2C, B7-H3, a ligand of CD83, and combinations thereof;
in some specific embodiments, the costimulatory signalling domain comprises a cytoplasmic domain of CD28 and/or a cytoplasmic domain of CD137;
in some specific embodiments, the costimulatory signalling domain comprises an amino acid sequence set forth in SEQ ID NO: 64, or an amino acid sequence having at least 80%, at least 85%, at least 90%, at least 95%, at least 96%, at least 97%, at least 98% or at least 99% identity to SEQ ID NO: 64.

In some embodiments of the present disclosure, in the chimeric antigen receptor, the intracellular signalling domain comprises a primary intracellular signalling domain;

in some specific embodiments, the primary intracellular signalling domain is derived from CD3$\zeta$;
in some specific embodiments, the primary intracellular signalling domain comprises an amino acid sequence set forth in SEQ ID NO: 65, or an amino acid sequence having at least 80%, at least 85%, at least 90%, at least 95%, at least 96%, at least 97%, at least 98% or at least 99% identity to SEQ ID NO: 65.

In some embodiments of the present disclosure, the chimeric antigen receptor has an amino acid sequence set forth in any one of the sequences of SEQ ID NO: 49 to SEQ ID NO: 53 or in any one of the sequences SEQ ID NO: 71 to SEQ ID NO: 75.
A further aspect of the present disclosure relates to an isolated nucleic acid, which encodes the chimeric antigen receptor according to any one in the present disclosure.
A further aspect of the present disclosure relates to a vector, which comprises the isolated nucleic acid of the present disclosure.
A further aspect of the present disclosure relates to a cell, which comprises the chimeric antigen receptor according to any one in the present disclosure, comprises the isolated nucleic acid of the present disclosure, or comprises the vector of the present disclosure;
in some specific embodiments, the cell is an immune cell.
In some embodiments of the present disclosure, the cell is selected from: a T cell, a NK cell, a peripheral blood mononuclear cell (PBMC), a hematopoietic stem cell, a pluripotent stem cell, an embryonic stem cell, and combinations thereof.
A further aspect of the present disclosure relates to a pharmaceutical composition, which comprises the anti-GCC antibody or the antigen-binding fragment thereof according to any one in the present disclosure, or the chimeric antigen receptor according to any one in the present disclosure, or the cell according to any one in the present disclosure;
in some specific embodiment, the pharmaceutical composition further comprises one or more pharmaceutically acceptable excipients.
A further aspect of the present disclosure relates to the use of the anti-GCC antibody or the antigen-binding fragment thereof according to any one in the present disclosure or the chimeric antigen receptor according to any one in the present disclosure in immunohistochemical detection or in the preparation of a detection kit.

A further aspect of the disclosure relates to the use of the anti-GCC antibody or the antigen-binding fragment thereof according to any one in the present disclosure, the chimeric antigen receptor according to any one in the present disclosure, or the cell according to one in the present disclosure in the preparation of a medicament for treating or preventing a GCC-related disease or condition;

in some specific embodiments, the GCC-related disease or condition is a tumour;
in some specific embodiments, the GCC-related disease or condition is a gastrointestinal tract tumour;
in some specific embodiments, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

The anti-GCC antibody or the antigen-binding fragment thereof according to any one in the present disclosure, the chimeric antigen receptor according to any one in the present disclosure, or the cell according to any one in the present disclosure is for use in treating or preventing a GCC-related disease or condition;

in some specific embodiments, the GCC-related disease or condition is a tumour;
in some specific embodiments, the GCC-related disease or condition is a gastrointestinal tract tumour;
in some specific embodiments, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

A further aspect of the present disclosure relates to a method for treating or preventing a GCC-related disease or condition, which method comprises the step of administering to a subject in need thereof an effective amount of the anti-GCC antibody or the antigen-binding fragment thereof according to any one in the present disclosure, the chimeric antigen receptor according to any one in the present disclosure, or the cell according to any one in the present disclosure;

in some specific embodiments, the GCC-related disease or condition is a tumour;
in some specific embodiments, the GCC-related disease or condition is a gastrointestinal tract tumour;
in some specific embodiments, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

In the present disclosure, the scientific and technical terms used herein have the meanings as commonly understood by those skilled in the art unless otherwise specified. Moreover, laboratory operation steps of cell culture, molecular genetics, nucleic acid chemistry, and immunology used herein are all conventional steps widely used in the corresponding art. Moreover, for a better understanding of the present disclosure, definitions and explanations of related terms are provided below.

As used herein, the term $EC_{50}$ refers to the concentration for 50% of maximal effect, i.e., the concentration that causes 50% of the maximal effect.

As used herein, the term "antibody" refers to an immunoglobulin molecule generally consisting of two pairs of polypeptide chains (each pair has one "light" (L) chain and one "heavy" (H) chain). Light chains of an antibody can be classified as $\kappa$ and $\lambda$ light chains. Heavy chains can be classified as $\mu$, $\delta$, $\gamma$, $\alpha$, or $\varepsilon$, and the isotypes of an antibody are defined as IgM, IgD, IgG, IgA, and IgE, respectively. Within light chains and heavy chains, the variable region and the constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of an antibody can mediate the binding of the immunoglobulin to host tissues or factors, including various cells of the immune system (e.g., effector cells) and the first component (C1q) of the classical complement system. VH and VL regions can be further subdivided into regions of hypervariability (termed complementarity determining regions (CDRs)), interspersed with more conserved regions termed framework regions (FRs). Each VH and VL consists of three CDRs and four FRs, arranged from the amino terminus to the carboxyl terminus in the following order: FR1, CDR1, FR2, CDR2, FR3, CDR3, and FR4.

The term "framework" or "FR" refers to those variable region residues flanking the CDRs. FR residues are those variable domain residues other than hypervariable region residues or CDR residues.

The terms "hypervariable region", "complementarity determining region", and "CDR" are used interchangeably.

"CDR" refers to one of the three hypervariable regions (H1, H2, or H3) within the non-framework regions of the immunoglobulin (Ig or antibody) VH β-sheet framework, or to one of the three hypervariable regions (L1, L2, or L3) within the non-framework regions of the antibody VL β-sheet framework. The positions of CDRs and FRs can be determined using a variety of definition methods well known in the art, such as, the Kabat numbering system, the AbM numbering system, the IMGT numbering system, the Chothia numbering system, or the Contact numbering system. The Kabat numbering system refers to the immunoglobulin alignment and numbering system proposed by Elvin A. Kabat (see, e.g., Kabat et al., Sequences of Proteins of Immunological Interest, 5th Ed. Public HealthService, National Institutes of Health, Bethesda, Md., 1991). The AbM numbering system uses an integrated suite of computer programs produced by Oxford Molecular Group that models antibody structures (see, e.g., Martin et al., 1989, ProcNatl Acad Sci (USA), 86: 9268-9272; "AbMTM, A Computer Program for ModelingVariable Regions of Antibodies," Oxford, UK; Oxford Molecular, Ltd). For the IMGT numbering system based on the international ImMunoGeneTics information system (IMGT) initiated by Lefranc et al., reference can be made to Lefranc et al., Dev. Comparat. Immunol. 27: 55-77, 2003. The Chothia numbering system is similar to the Kabat numbering system, but the Chothia numbering system contemplates the positions of certain structural loop regions. (See, e.g., Chothia et al., 1986, J. Mol. Biol., 196: 901-17; Chothia et al., 1989, Nature, 342: 877-83). The Contact numbering system is based on the analysis of available complex crystal structures (see, e.g., MacCallum et al., 1996, J. Mol. Biol., 5: 732-745). Herein, CDRs are determined on the basis of amino acid sequences and their positions within the light chains or heavy chains. Since the "positions" of CDRs within an immunoglobulin variable region domain are conserved across species, the amino acid residues of the CDRs and framework regions can be readily identified by aligning the amino acid sequence of a variable region domain with the structural features delineated using a known numbering system. Amino acid residues from each of these hypervariable regions or CDRs are exemplified in Table 1 below.

**Table 1. Exemplary CDRs according to various numbering** systems

| CDR | Kabat | AbM | Chothia | Contact | IMGT |
|---|---|---|---|---|---|
| L-CDR1 | L24--L34 | L24--L34 | L26--L32 or L24--L34 | L30--L36 | L27--L38 |
| L-CDR2 | L50--L56 | L50--L56 | L50--L52 or L50--L56 | L46--L55 | L56--L65 |
| L-CDR3 | L89--L97 | L89--L97 | L91--L96 or L89--L97 | L89--L96 | L105-L117 |
| H-CDR1 | H31--H35B (Kabat numbering) | H26--H35B | H26--H32..34 | H30--H35B | H27--H38 |
| H-CDR1 | H31--H35 (Chothia numbering) | H26--H35 | H26--H32 | H30--H35 | |
| H-CDR2 | H50--H65 | H50--H58 | H53--H55 or H52--H56 | H47--H58 | H56--H65 |
| H-CDR3 | H95--H102 | H95--H102 | H96--H101 or H95--H102 | H93--H101 | H105-H117 |

The boundaries of CDRs may vary depending on the numbering method used. Thus, unless otherwise specified, the terms "CDR" and "complementarity determining region" of a given antibody or a region thereof (e.g., a variable region), as well as an individual CDR of the antibody or the region thereof (e.g., HCDR1, HCDR2), should be understood to encompass complementarity determining regions as defined by any known scheme described above. In some cases, a scheme designated for identifying a particular CDR is provided, e.g., a CDR defined by the IMGT, Kabat, AbM, Chothia, or Contact method. In other cases, a particular amino acid sequence of a CDR is provided. It should be noted that CDRs can also be determined by a combination of various numbering systems, such as a combination of the Kabat and Chothia numbering systems, a combination of the Kabat and AbM numbering systems, or a combination of the Kabat and IMGT numbering systems. Thus, a term such as "CDR1 as set forth in a particular VH or VL" includes, for example, any CDR1 as defined by the exemplary CDR numbering systems described above, but is not limited thereto. Once the amino acid sequence of a variable region (e.g., VH or VL) is given, those skilled in the art will understand that the CDRs within that region can be defined by different numbering systems or combinations thereof.

The term "antibody" is not limited by any particular method for producing an antibody. For example, the antibody includes a recombinant antibody, a monoclonal antibody, and a polyclonal antibody. The antibody may be antibodies of different isotypes, e.g., an IgG (e.g., IgG1, IgG2, IgG3, or IgG4 subtype), IgA1, IgA2, IgD, IgE, or IgM antibody.

As used herein, the terms "monoclonal antibody" and "mAb" refer to an antibody or a fragment of an antibody from a population of highly homologous antibody molecules, i.e., a population of identical antibody molecules, except for possible naturally occurring mutations. The monoclonal antibody is highly specific for a single epitope on an antigen. A polyclonal antibody, in contrast to a monoclonal antibody, typically comprises at least two or more different antibodies, which usually recognise different epitopes on an antigen. Monoclonal antibodies can usually be obtained using the hybridoma technology first reported by Kohler et al. (Köhler G, Milstein C. Continuous cultures of fused cells secreting antibody of predefined specificity [J]. nature, 1975; 256 (5517): 495), but can also be obtained using recombinant DNA technology (see, e.g., U.S. Patent 4,816,567).

As used herein, the term "humanised antibody" refers to an antibody or antibody fragment obtained by replacing all or part of the CDR regions of a human immunoglobulin (recipient antibody) with the CDR regions of a non-human antibody (donor antibody), wherein the donor antibody can be a non-human (e.g., mouse, rat, or rabbit) antibody with the desired specificity, affinity, or reactivity. Furthermore, some amino acid residues in the framework regions (FRs) of the recipient antibody can also be replaced with corresponding amino acid residues of the non-human antibody, or with amino acid residues of other antibodies, to further improve or optimise the performance of the antibody. For more details regarding humanised antibodies, reference can be made to, for example, Jones et al., Nature 1986; 321: 522 525; Reichmann et al., Nature, 1988; 332: 323 329; Presta, Curr. Op. Struct. Biol. 1992; 2: 593-596; and Clark, Immunol. Today 2000; 21: 397 402. In some cases, antigen-binding fragments of antibodies are diabodies, in which VH and VL domains are expressed on a single polypeptide chain but with a linker that is too short to allow pairing between the two domains on the same chain, thereby forcing the domains to pair with the complementary domains of another chain and generating two antigen-binding sites (see, e.g., Holliger P. et al., Proc. Natl. Acad. Sci. USA 1993; 90: 6444-6448 and Poljak R. J. et al., Structure 1994; 2: 1121-1123).

As used herein, the term "single chain fragment variable (ScFv)" refers to a molecule comprising an antibody heavy chain variable region ($V_H$) and an antibody light chain variable region ($V_L$) linked by a linker. The $V_L$ and $V_H$ domains are paired via a linker which enables them to be produced as a single polypeptide chain, thereby forming a monovalent molecule (see, e.g., Bird et al, Science 1988; 242: 423-426 and Huston et al, Proc. Natl. Acad. Sci. USA 1988; 85: 5879-5883). Such scFv molecules may have a general structure: $NH2-V_L$-linker fragment-$V_H$-COOH or $NH2-V_H$-linker fragment-$V_L$-COOH. Suitable linkers in the prior art consist of repeated GGGGS (SEQ ID NO: 69) amino acid sequences or variants thereof. For example, a linker having an amino acid sequence (GGGGS)4 (SEQ ID NO: 70) may be used, and a variant thereof may also be used (Holliger et al, (1993), Proc. Natl. Acad. Sci. USA 90: 6444-6448). Other linkers that can be used in the present disclosure are described by Alfthan et al, Protein Eng. 1995; 8: 725-731, Choi et al, Eur. J. Immunol. 2001; 31: 94-106, Hu et al, Cancer Res. 1996; 56: 3055-3061, Kipriyanov et al, J. Mol. Biol. 1999; 293: 41-56, and Roovers et al, Cancer Immunology, Immunotherapy, 2001, 50 (1): 51-59.

As used herein, the term "isolated" or "being isolated" refers to that which is obtained from its natural state by artificial means. If a certain "isolated" substance or component occurs in nature, it may be because its natural environment has changed, or because it has been separated from the natural environment, or because both have occurred. For example, an unisolated polynucleotide or polypeptide is naturally present in a living animal, and the same polynucleotide or polypeptide isolated in high purity from this natural state is referred to as isolated. The term "isolated" or "being isolated" does not exclude the presence of artificial or synthetic substances, nor does it exclude the presence of other impure substances that do not affect the activity of the substance.

As used herein, the term "vector" refers to a nucleic acid delivery vehicle into which a polynucleotide can be inserted. When the vector allows for the expression of the protein encoded by the inserted polynucleotide, the vector is called an expression vector. A vector can be introduced into a host cell by transformation, transduction or transfection, and the genetic substance elements carried thereby can be expressed in the host cell. Vectors are well known to those skilled in the art, and include, but are not limited to: plasmids; phagemids; cosmids; artificial chromosomes, such as yeast artificial chromosomes (YACs), bacterial artificial chromosomes (BACs) or P1-derived artificial chromosomes (PACs); phages, such as λ phages or M13 phages and animal viruses. The animal viruses that can be used as vectors include, but are not limited to, retroviruses (including lentiviruses), adenoviruses, adeno-associated viruses, herpes viruses (e.g., herpes simplex virus), poxviruses, baculoviruses, papilloma viruses, and papovaviruses (such as SV40). A vector may comprise a variety of elements that control expression, including but not limited to a promoter sequence, a transcription initiation sequence, an enhancer sequence, a selection element, and a reporter gene. In addition, the vector may further comprise an origin of replication.

As used herein, the term "host cell" refers to a cell that can be used for vector introduction, including but not limited to a prokaryotic cell such as *Escherichia coli* or *Bacillus subtilis,* a fungal cell such as a yeast cell or *Aspergillus,* an insect cell such as *Drosophila* S2 cell or Sf9, or an animal cell such as a fibroblast, a CHO cell, a GS cell, a COS cell, an NSO cell, a HeLa cell, a BHK cell, an HEK 293 cell, or a human cell.

As used herein, the term "specific binding" refers to a non-random binding reaction between two molecules, such as a reaction between an antibody and its corresponding antigen. In certain embodiments, an antibody specifically binding to an antigen (or an antibody specific for an antigen) refers to an antibody that binds to the antigen with an affinity ($K_D$) of less than about $10^{-5}$ M, for example, less than about $10^{-6}$ M, $10^{-7}$ M, $10^{-8}$ M, $10^{-9}$ M, or $10^{-10}$ M, or less.

As used herein, the term "$K_D$" refers to the equilibrium dissociation constant of a particular antibody-antigen interaction, which is used to describe the binding affinity between an antibody and an antigen. The smaller the equilibrium dissociation constant, the tighter the antibody-antigen binding and the higher the affinity between the antibody and the antigen. Generally, an antibody binds to an antigen (e.g., a GCC protein) with an equilibrium dissociation constant ($K_D$) of less than about $10^{-5}$ M, for example, less than about $10^{-6}$ M, $10^{-7}$ M, $10^{-8}$ M, $10^{-9}$ M, or $10^{-10}$ M, or less. $K_D$ can be determined using methods known to those skilled in the art, for example, by determination using a Fortebio molecular interaction instrument.

As used herein, the terms "monoclonal antibody" and "mAb" have the same meaning and are used interchangeably; The terms "polyclonal antibody" and "pAb" have the same meaning and are used interchangeably. Moreover, in the present disclosure, amino acids are generally represented by single-letter and three-letter abbreviations well known in the art. For example, alanine can be represented by A or Ala.

As used herein, the term "immunohistochemistry" or "IHC" refers to an immunocytochemical study conducted at the tissue level. "Immunohistochemical staining" is a type of immunostaining in which a fluorescent or chromogenic chemical substance is bound to an antibody. Utilising the immunological principle that such a modified antibody specifically binds to an antigen in biological tissue, it selectively recognises antigens in tissue sections or smear cells, thereby detecting the presence or absence of a target antigen in cells or tissues. This method can not only be used to detect the expression level of an antigen but also to observe the localisation of antigen expression, thereby enhancing the sensitivity and specificity of pathological diagnosis. In immunohistochemical staining, specific antibodies that have been artificially prepared and modified are labelled with a developer, such as enzymes, metal ions, and isotopes; the target antigen to be detected in cells or tissues can be any substance to which an antibody can bind, i.e., an antigenic substance, including a protein, a polypeptide, a nucleic acid, a polysaccharide, a pathogen, etc. The two enable the localisation, qualitative analysis and quantitative analysis of antigens in tissues or cells through the immunological antigen-antibody reaction, the histochemical chromogenic reaction, in conjunction with the morphological analysis of tissue cells.

As used herein, the "chimeric antigen receptor" or "CAR" refers to a genetically engineered receptor that can be used to graft specificity for one or more antigens onto an immune cell, such as a T cell. Some CARs are also known as "artificial T cell receptors", "chimeric T cell receptors", or "chimeric immune receptors". In some embodiments, the CAR comprises an extracellular antigen-binding domain specific for one or more antigens (e.g., tumour antigens), a transmembrane domain, and an intracellular signalling domain of a T cell and/or other receptors. The "CAR-T cell" refers to a T cell that expresses a CAR.

The terms "subject" and "patient" are used interchangeably herein. As used herein, in certain embodiments, the subject is a mammal, such as a non-primate or a primate (e.g., a human). The subject can be a mammal, such as a human, who has been diagnosed with a disease or condition. The subject can be a mammal, such as a human, who is at risk of developing a disease or condition.

As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to: a pH regulator, a surfactant, an adjuvant, an ionic strength enhancer. For example, the pH regulator includes, but is not limited to, a phosphate buffer; the surfactant includes, but is not limited to, a cationic, anionic, or nonionic surfactant, such as Tween-80; and the ionic strength enhancer includes, but is not limited to, sodium chloride.

As used herein, the term "effective amount" refers to an amount sufficient to obtain or at least partially obtain the desired effect. For example, an effective amount for preventing a disease (e.g., a tumour) refers to an amount sufficient to prevent, inhibit or delay the onset of a disease (e.g., a tumour); an effective amount for treating a disease refers to an amount sufficient to cure or at least partially inhibit a disease and complications thereof in a patient suffering from the disease. It is well within the competence of those skilled in the art to determine such effective amounts. For example, an amount effective for therapeutic use will depend on the severity of the disease to be treated, the overall status of the patient's own immune system, the general situation of the patient such as age, body weight and gender, the mode of administration of the drug, other concurrent treatments, etc.

**Beneficial Effects**

The present disclosure achieves one or more of the following technical effects:

(1) The anti-GCC antibody or the antigen-binding fragment thereof of the present disclosure has good specificity.
(2) The anti-GCC antibody or the antigen-binding fragment thereof of the present disclosure has high affinity.
(3) The anti-GCC antibody or the antigen-binding fragment thereof of the present disclosure has high sensitivity in immunohistochemical analysis and has good application prospects in tumour detection and in the preparation of detection kits.
(4) The anti-GCC antibody or the antigen-binding fragment thereof of the present disclosure, the chimeric antigen receptor of the present disclosure, or the chimeric antigen receptor T cell of the present disclosure has good potential for treating or preventing tumours.

**Brief Description of the Drawings**

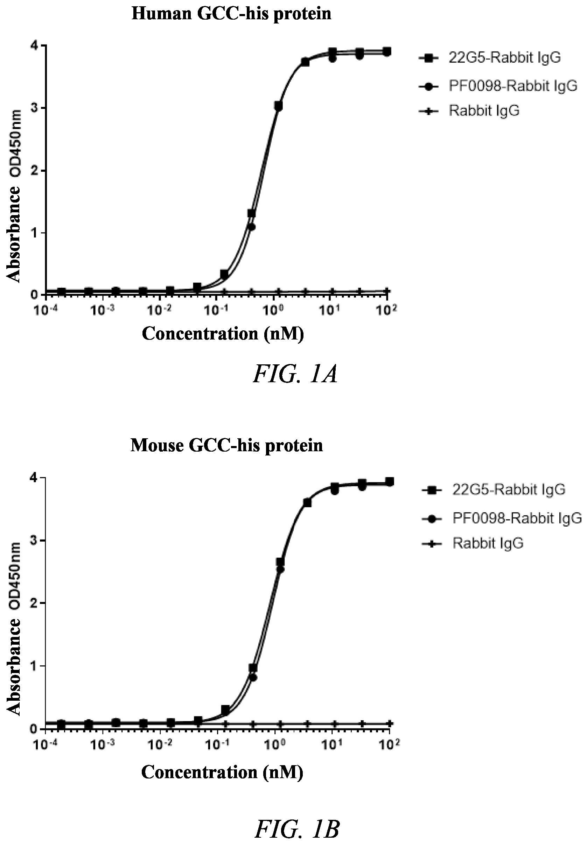

FIG. 1A to FIG. 1B show the binding curves of anti-GCC antibody 22G5-Rabbit IgG to target proteins human GCC-his protein (FIG. 1A) and mouse GCC-his protein (FIG. 1B), as detected by enzyme-linked immunosorbent assay, with PF0098-Rabbit IgG serving as the control. The binding of the 22G5-Rabbit IgG to the GCC target protein is concentration-dependent, and its $EC_{50}$ is comparable to that of the PF0098-Rabbit IgG antibody.

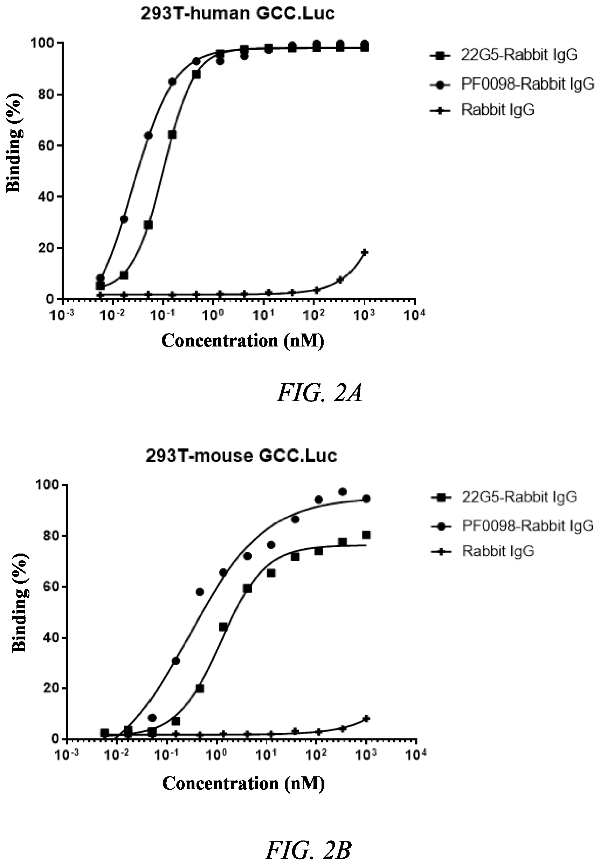

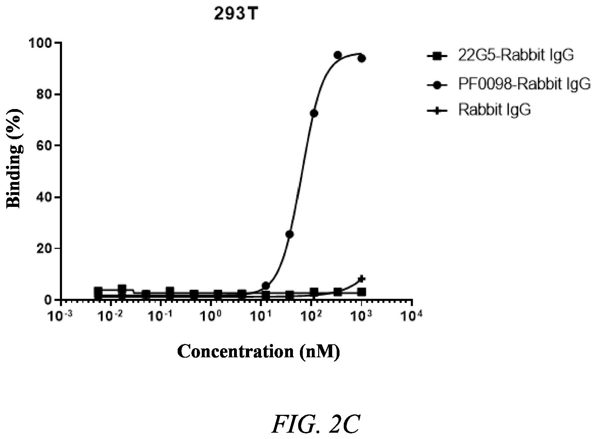

FIG. 2A to FIG. 2C show the binding curves of anti-GCC antibody 22G5-Rabbit IgG to target cells, as detected by flow cytometry, with PF0098-Rabbit IgG serving as the control. The binding of the 22G5-Rabbit IgG to the target cells 293T-human GCC.Luc (FIG. 2A) and 293T-mouse GCC.Luc (FIG. 2B) is concentration-dependent, and no non-specific binding to 293T cells (FIG. 2C) is observed, with specificity superior to that of PF0098-Rabbit IgG. Therefore, 22G5-Rabbit IgG is a novel anti-GCC antibody with high specificity.

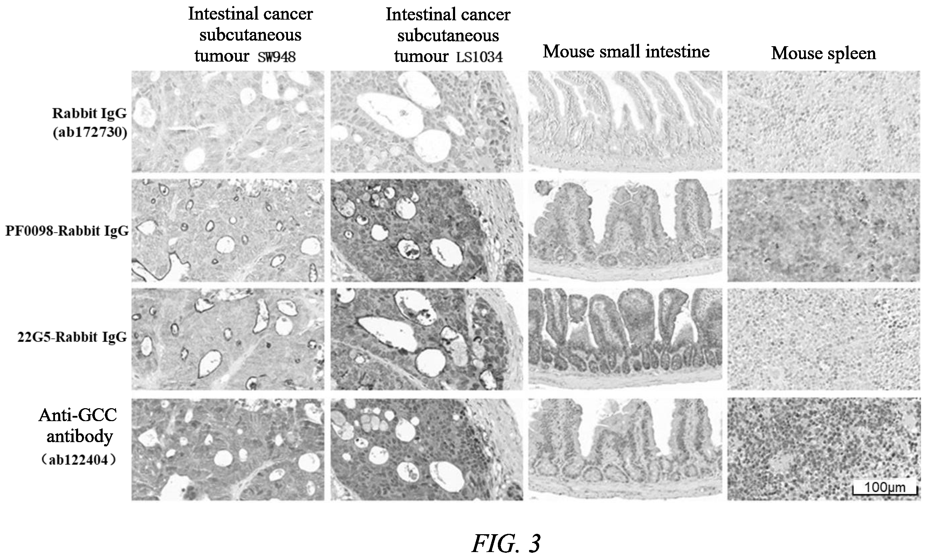

FIG. 3 shows experimental results of immunohistochemical detection of tissue sections of subcutaneous tumours derived from human intestinal cancer cell line SW948, human intestinal cancer cell line LS1034, mouse small intestine, and mouse spleen, using different anti-GCC antibodies at the same concentration (0.5 $\mu$g/mL). The isotype control antibody ab172730 shows no staining in any of the four tissue sections. The PF0098-Rabbit IgG antibody exhibits strong GCC-specific staining in the sections of SW948 subcutaneous tumour and LS1034 subcutaneous tumour, and produces non-specific staining in mouse spleen. The 22G5-Rabbit IgG antibody exhibits strong GCC-specific staining in the sections of SW948 subcutaneous tumour, LS1034 subcutaneous tumour, and mouse small intestine; and its cell membrane staining intensity is higher than that of the PF0098-Rabbit IgG antibody, and no non-specific staining is observed in any of the four tissues. The anti-GCC antibody ab122404 exhibits cytoplasm-localised staining in the sections of SW948 subcutaneous tumour and LS1034 subcutaneous tumour, shows no GCC-specific staining on the cell membrane surface, and produces non-specific staining in the mouse small intestine and the mouse spleen. Therefore, for immunohistochemical staining applications, the 22G5-Rabbit IgG antibody has superior specificity and sensitivity.

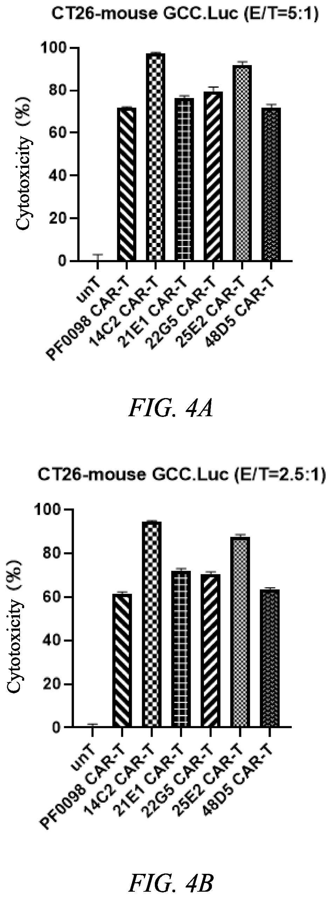

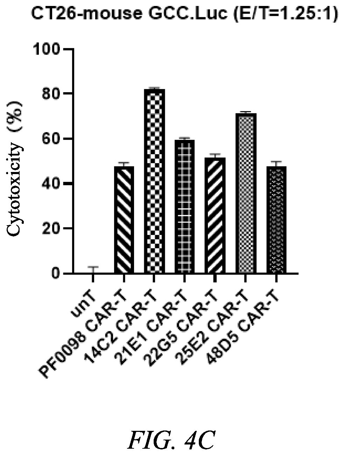

FIG. 4A to FIG. 4C show the cytotoxicity results of the constructed CAR-T cells against target cells CT26-mouse GCC.Luc overexpressing mouse GCC at effector-to-target ratios of 5 : 1 (FIG. 4A), 2.5 : 1 (FIG. 4B), and 1.25 : 1 (FIG. 4C), respectively, with PF0098 CAR-T serving as the positive control. All CAR-T cells effectively kill mouse GCC-positive target cells.

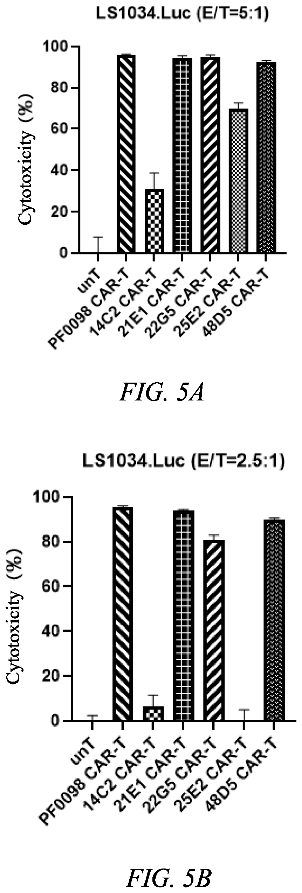

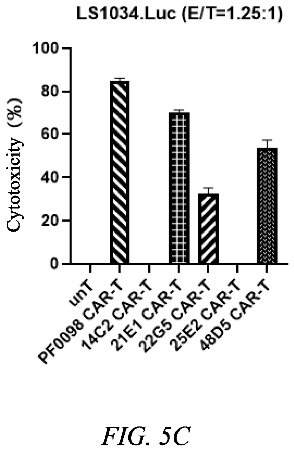

FIG. 5A to FIG. 5C show the cytotoxicity results of the constructed CAR-T cells against target cells LS1034.Luc natively expressing human GCC at effector-to-target ratios of 5 : 1 (FIG. 5A), 2.5 : 1 (FIG. 5B), and 1.25 : 1 (FIG. 5C), respectively, with PF0098 CAR-T serving as the control. 14C2 CAR-T, which recognises mouse targets, does not effectively kill the target cells, whereas the remaining CAR-T cells exhibit potent cytotoxicity against human GCC-positive target cells. This indicates that rabbit-derived scFvs are also applicable to the antigen-binding domain of CARs and enable the CAR-T cells to exert highly efficient recognition and cytotoxicity functions.

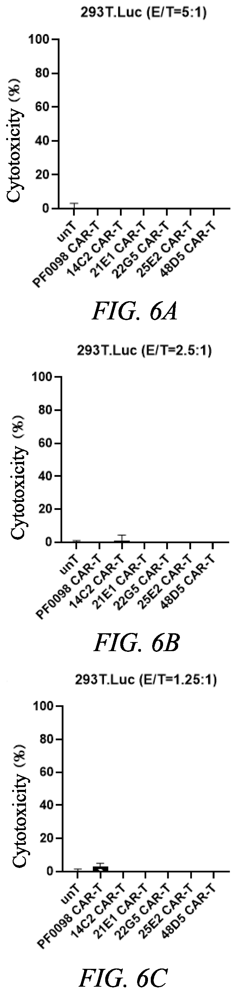

FIG. 6A to FIG. 6C show the cytotoxicity of the constructed CAR-T cells against 293T.Luc cells without GCC expression at effector-to-target ratios of 5 : 1 (FIG. 6A), 2.5 : 1 (FIG. 6B), and 1.25 : 1 (FIG. 6C), respectively. All CAR-Ts fail to kill 293T.Luc, indicating that the constructed CAR-T cells only specifically recognise GCC-positive cells.

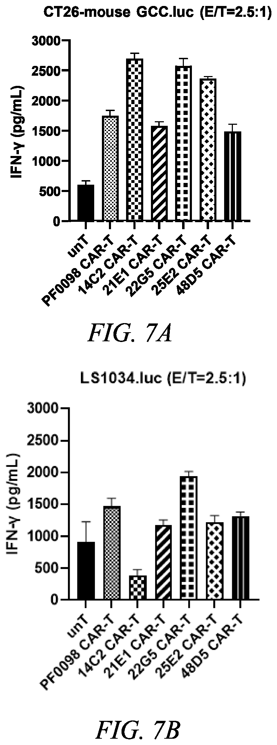

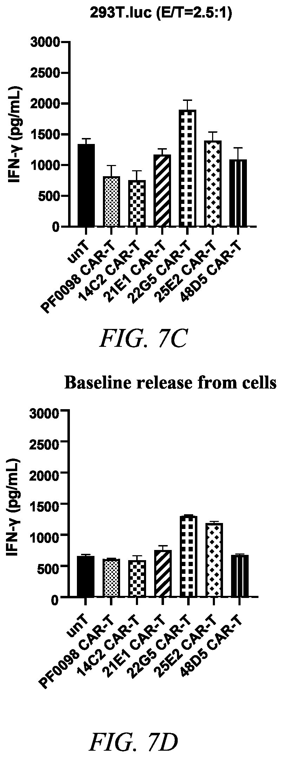

FIG. 7A to FIG. 7D show cytokine secretion levels of the constructed CAR-T cells after co-incubation with target cells CT26-mouse GCC.Luc (FIG. 7A), LS1034.Luc (FIG. 7B), and 293T.Luc (FIG. 7C) for 24 hours, respectively. Following co-incubation with the target cells CT26-mouse GCC.Luc or LS1034.Luc, IFN$\gamma$ secretion by the CAR-T cells is upregulated. FIG. 7D shows the baseline IFN$\gamma$ secretion levels of UnT cells and each group of CAR-T cells under the condition of no co-culture with target cells.

Some of the sequences involved in the present disclosure are as follows.

SEQ ID NO: 1 14C2 heavy chain variable region CDR1
NYDMS
SEQ ID NO: 2 21E1 heavy chain variable region CDR1
SYDMT
SEQ ID NO: 3 22G5 heavy chain variable region CDR1
SYAMT
SEQ ID NO: 4 25E2 heavy chain variable region CDR1
KHAMG
SEQ ID NO: 5 48D5 heavy chain variable region CDR1
SNAIS
SEQ ID NO: 6 14C2 heavy chain variable region CDR2

TIEPDGSTWYASWAKG

SEQ ID NO: 7 21E1 heavy chain variable region CDR2
SIGDDGNTYYATWAKG

SEQ ID NO: 8 22G5 heavy chain variable region CDR2
TINTISTAYYANWAKG

SEQ ID NO: 9 25E2 heavy chain variable region CDR2
TIYVSTNTAYASWAKG

SEQ ID NO: 10 48D5 heavy chain variable region CDR2
YIGTGGKSFYATWTKG

SEQ ID NO: 11 14C2 heavy chain variable region CDR3
GAPDYSSNAFNL

SEQ ID NO: 12 21E1 heavy chain variable region CDR3
GWSFCKS

SEQ ID NO: 13 22G5 heavy chain variable region CDR3
DHGYYDV

SEQ ID NO: 14 25E2 heavy chain variable region CDR3
GGSSYYTGELYFNL

SEQ ID NO: 15 48D5 heavy chain variable region CDR3
GNTYLDL

SEQ ID NO: 16 14C2 light chain variable region CDR1
QASQSISSYLA

SEQ ID NO: 17 21E1 light chain variable region CDR1
QSSQSVANNNLA

SEQ ID NO: 18 22G5 light chain variable region CDR1
QASQSVYKNNYLS

SEQ ID NO: 19 25E2 light chain variable region CDR1
QVSQSIGSWLA

SEQ ID NO: 20 48D5 light chain variable region CDR1
QSSQSVYGADYLA

SEQ ID NO: 21 14C2 light chain variable region CDR2
KASTLAS

SEQ ID NO: 22 21E1 light chain variable region CDR2
KASTLES

SEQ ID NO: 23 22G5 light chain variable region CDR2
DASTLAS

SEQ ID NO: 24 25E2 light chain variable region CDR2
SASTLAS

SEQ ID NO: 25 48D5 light chain variable region CDR2
RASNLAS

SEQ ID NO: 26 14C2 light chain variable region CDR3
QSTYAASNKLHFA

SEQ ID NO: 27 21E1 light chain variable region CDR3
QGAYRSGIMA

SEQ ID NO: 28 22G5 light chain variable region CDR3
LGEFSCSSGDCAA

SEQ ID NO: 29 25E2 light chain variable region CDR3
QSYSSASTTA

SEQ ID NO: 30 48D5 light chain variable region CDR3
QGTYISSDWLNP

SEQ ID NO: 31 14C2 heavy chain variable region

QSLEESGGRLVTPGTPLTLTCTASGFSLSNYDMSWVRQAPGKGLEWI
GTIEPDGSTWYASWAKGRLTISRTSTTVDLKMTSLTTEDTATYFCVRGAPD
YSSNAFNLWGQGTLVTVSS

SEQ ID NO: 32 21E1 heavy chain variable region

QSLEESGGRLVTPGTPLTLTCTVSGIDLSSYDMTWVRQAPGKGLEWIG
SIGDDGNTYYATWAKGRFTISKTSTTVHLKMTSLTTEDTATYFCARGWSF
CKSWGQGTLVTVSS

SEQ ID NO: 33 22G5 heavy chain variable region

QSVEESGGRLVTPGTPLTLTCTVSGIDLSSYAMTWVRQAPGEGLEWIG
TINTISTAYYANWAKGRFTISKTSTTVDLKIASPTTEDTATYFCARDHGYY
DVWGQGTLVTVSS

SEQ ID NO: 34 25E2 heavy chain variable region

QSVEESGGRLVTPGTPLTLTCTVSGIDLNKHAMGWVRQAPTKGLEWI
GTIYVSTNTAYASWAKGRFTISKTSSTTVDLKMTSLTTEDTATYFCARGGG
SSYYTGELYFNLWGQGTLVTVSS

SEQ ID NO: 35 48D5 heavy chain variable region

QSLEESGGRLVKPDETLTITCTVSGIDLSSNAISWVRQAPGEGLEYIGYI
GTGGKSFYATWTKGRFTISKTSTTVDLKMTSLTNEDTAMYFCATGNTYLD
LWGQGTLVTVSS

SEQ ID NO: 36 PF0098 heavy chain variable region

QVQLQQPGAELVKPGASVKLSCKASGYTFTSYWMHWVKQRPGQGL
EWIGEIKPSNGLTNYIEKFKNKATLTVDKSATTAYMQLSSLTAEDSAVYYC
TRTITTTEGYWFFDVWGAGTTVTVSS

SEQ ID NO: 37 14C2 light chain variable region

DVVMTQTPSSVSEPVGGTVTIKCQASQSISSYLAWYQQKPGQPPKLLI
YKASTLASGVSSQFRGSGSGTEFTLTISDLECADAATYYCQSTYAASNKLH
FAFGGGTDVVK

SEQ ID NO: 38 21E1 light chain variable region

AQVLTQTASPVSAAVGGTVTISCQSSQSVANNNLAWYQQKPGQPPSP
LIYKASTLESGVPSRFSGSGSGTQFALTISGVQCDDAATYYCQGAYRSGIM
AFGGGTEVVVR

SEQ ID NO: 39 22G5 light chain variable region

AQVLTQTPSSVSAAVGGTVTINCQASQSVYKNNYLSWYQQKPGQPPR
LLIYDASTLASGVPSRFKGSGSGTQFTLTISGVQCDDAATYYCLGEFSCSSG
DCAAFGGGTEVVVK

SEQ ID NO: 40 25E2 light chain variable region

AYDMTQTPASVEVAVGGTVSIKCQVSQSIGSWLAWYQQKPGQPPKL
LIYSASTLASGVSSRFKGSGSGTEFTLTISDLACVDAATYYCQSYSSASTTA
FGGGTEVVVR

SEQ ID NO: 41 48D5 light chain variable region

AQVLTQTPSSVSAAVGGTVTINCQSSQSVYGADYLAWYQQKPGQPPK
LLIYRASNLASGVPSRFRGSGSGTQFTLTISEVQCDDAATYYCQGTYISSDW
LNPFGGGTEVVVK

SEQ ID NO: 42 PF0098 light chain variable region

DIVLTQSPASLAVSLGQRATISCRASESVDYYGTSLMQWYQQKPGQPP
KLLIYAASNVESGVPARFSGSGSGTDFSLNIHPVEEDDIAMYFCQQTRKVY
TFGGGTKLEIK

SEQ ID NO: 43 14C2 single chain variable fragment

DVVMTQTPSSVSEPVGGTVTIKCQASQSISSYLAWYQQKPGQPPKLLI
YKASTLASGVSSQFRGSGSGTEFTLTISDLECADAATYYCQSTYAASNKLH
FAFGGGTDVVVKGGGGSGGGGSGGGGSQSLEESGGRLVTPGTPLTLTCTA
SGFSLSNYDMSWVRQAPGKGLEWIGTIEPDGSTWYASWAKGRLTISRTST
TVDLKMTSLTTEDTATYFCVRGAPDYSSNAFNLWGQGTLVTVSS

SEQ ID NO: 44 21E1 single chain variable fragment

AQVLTQTASPVSAAVGGTVTISCQSSQSVANNNLAWYQQKPGQPPSP
LIYKASTLESGVPSRFSGSGSGTQFALTISGVQCDDAATYYCQGAYRSGIM
AFGGGTEVVVRGGGGSGGGGSGGGGSQSLEESGGRLVTPGTPLTLTCTVS
GIDLSSYDMTWVRQAPGKGLEWIGSIGDDGNTYYATWAKGRFTISKTSTT
VHLKMTSLTTEDTATYFCARGWSFCKSWGQGTLVTVSS

SEQ ID NO: 45 22G5 single chain variable fragment

AQVLTQTPSSVSAAVGGTVTINCQASQSVYKNNYLSWYQQKPGQPPR
LLIYDASTLASGVPSRFKGSGSGTQFTLTISGVQCDDAATYYCLGEFSCSSG
DCAAFGGGTEVVVKGGGGSGGGGSGGGGSQSVEESGGRLVTPGTPLTLTC
TVSGIDLSSYAMTWVRQAPGEGLEWIGTINTISTAYYANWAKGRFTISKTS
TTVDLKIASPTTEDTATYFCARDHGYYDVWGQGTLVTVSS

SEQ ID NO: 46 25E2 single chain variable fragment

AYDMTQTPASVEVAVGGTVSIKCQVSQSIGSWLAWYQQKPGQPPKL
LIYSASTLASGVSSRFKGSGSGTEFTLTISDLACVDAATYYCQSYSSASTTA
FGGGTEVVVRGGGGSGGGGSGGGGSQSVEESGGRLVTPGTPLTLTCTVSG
IDLNKHAMGWVRQAPTKGLEWIGTIYVSTNTAYASWAKGRFTISKTSSTT
VDLKMTSLTTEDTATYFCARGGGSSYYTGELYFNLWGQGTLVTVSS

SEQ ID NO: 47 48D5 single chain variable fragment

AQVLTQTPSSVSAAVGGTVTINCQSSQSVYGADYLAWYQQKPGQPPK
LLIYRASNLASGVPSRFRGSGSGTQFTLTISEVQCDDAATYYCQGTYISSDW
LNPFGGGTEVVVKGGGGSGGGGSGGGGSQSLEESGGRLVKPDETLTITCT
VSGIDLSSNAISWVRQAPGEGLEYIGYIGTGGKSFYATWTKGRFTISKTSTT
VDLKMTSLTNEDTAMYFCATGNTYLDLWGQGTLVTVSS

SEQ ID NO: 48 PF0098 single chain variable fragment

DIVLTQSPASLAVSLGQRATISCRASESVDYYGTSLMQWYQQKPGQPP
KLLIYAASNVESGVPARFSGSGSGTDFSLNIHPVEEDDIAMYFCQQTRKVY
TFGGGTKLEIKGSTSGSGKPGSGEGSTKGQVQLQQPGAELVKPGASVKLSC
KASGYTFTSYWMHWVKQRPGQGLEWIGEIKPSNGLTNYIEKFKNKATLTV
DKSATTAYMQLSSLTAEDSAVYYCTRTITTTEGYWFFDVWGAGTTVTVSS

SEQ ID NO: 49 14C2 CAR

MALPVTALLLPLALLLHAARPDVVMTQTPSSVSEPVGGTVTIKCQAS
QSISSYLAWYQQKPGQPPKLLIYKASTLASGVSSQFRGSGSGTEFTLTISDL
ECADAATYYCQSTYAASNKLHFAFGGGTDVVVKGGGGSGGGGSGGGGS
QSLEESGGRLVTPGTPLTLTCTASGFSLSNYDMSWVRQAPGKGLEWIGTIE
PDGSTWYASWAKGRLTISRTSTTVDLKMTSLTTEDTATYFCVRGAPDYSS
NAFNLWGQGTLVTVSSTTTPAPRPPTPAPTIASQPLSLRPEACRPAAGGAV
HTRGLDFACDIYIWAPLAGTCGVLLLSLVITLYCKRGRKKLLYIFKQPFMR
PVQTTQEEDGCSCRFPEEEEGGCELRVKFSRSADAPAYQQGQNQLYNELN
LGRREEYDVLDKRRGRDPEMGGKPRRKNPQEGLYNELQKDKMAEAYSEI
GMKGERRRGKGHDGLYQGLSTATKDTYDALHMQALPPR

SEQ ID NO: 50 21E1 CAR

MALPVTALLLPLALLLHAARPAQVLTQTASPVSAAVGGTVTISCQSSQ
SVANNNLAWYQQKPGQPPSPLIYKASTLESGVPSRFSGSGSGTQFALTISGV
QCDDAATYYCQGAYRSGIMAFGGGTEVVVRGGGGSGGGGSGGGGSQSLE
ESGGRLVTPGTPLTLTCTVSGIDLSSYDMTWVRQAPGKGLEWIGSIGDDGN
TYYATWAKGRFTISKTSTTVHLKMTSLTTEDTATYFCARGWSFCKSWGQ

GTLVTVSSTTTPAPRPPTPAPTIASQPLSLRPEACRPAAGGAVHTRGLDFAC
DIYIWAPLAGTCGVLLLSLVITLYCKRGRKKLLYIFKQPFMRPVQTTQEED
GCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRG
KGHDGLYQGLSTATKDTYDALHMQALPPR

SEQ ID NO: 51 22G5 CAR

MALPVTALLLPLALLLHAARPAQVLTQTPSSVSAAVGGTVTINCQAS
QSVYKNNYLSWYQQKPGQPPRLLIYDASTLASGVPSRFKGSGSGTQFTLTI
SGVQCDDAATYYCLGEFSCSSGDCAAFGGGTEVVVKGGGGSGGGGSGGG
GSQSVEESGGRLVTPGTPLTLTCTVSGIDLSSYAMTWVRQAPGEGLEWIGT
INTISTAYYANWAKGRFTISKTSTTVDLKIASPTTEDTATYFCARDHGYYD
VWGQGTLVTVSSTTTPAPRPPTPAPTIASQPLSLRPEACRPAAGGAVHTRG
LDFACDIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRR
EEYDVLDKRRGRDPEMGGKPRRKNPQEGLYNELQKDKMAEAYSEIGMK
GERRRGKGHDGLYQGLSTATKDTYDALHMQALPPR

SEQ ID NO: 52 25E2 CAR

MALPVTALLLPLALLLHAARPAYDMTQTPASVEVAVGGTVSIKCQVS
QSIGSWLAWYQQKPGQPPKLLIYSASTLASGVSSRFKGSGSGTEFTLTISDL
ACVDAATYYCQSYSSASTTAFGGGTEVVVRGGGGSGGGGSGGGGSQSVE
ESGGRLVTPGTPLTLTCTVSGIDLNKHAMGWVRQAPTKGLEWIGTIYVST
NTAYASWAKGRFTISKTSSTTVDLKMTSLTTEDTATYFCARGGGSSYYTG
ELYFNLWGQGTLVTVSSTTTPAPRPPTPAPTIASQPLSLRPEACRPAAGGAV
HTRGLDFACDIYIWAPLAGTCGVLLLSLVITLYCKRGRKKLLYIFKQPFMR
PVQTTQEEDGCSCRFPEEEEGGCELRVKFSRSADAPAYQQGQNQLYNELN
LGRREEYDVLDKRRGRDPEMGGKPRRKNPQEGLYNELQKDKMAEAYSEI
GMKGERRRGKGHDGLYQGLSTATKDTYDALHMQALPPR

SEQ ID NO: 53 48D5 CAR

MALPVTALLLPLALLLHAARPAQVLTQTPSSVSAAVGGTVTINCQSSQ
SVYGADYLAWYQQKPGQPPKLLIYRASNLASGVPSRFRGSGSGTQFTLTIS
EVQCDDAATYYCQGTYISSDWLNPFGGGTEVVVKGGGGSGGGGSGGGGS
QSLEESGGRLVKPDETLTITCTVSGIDLSSNAISWVRQAPGEGLEYIGYIGTG
GKSFYATWTKGRFTISKTSTTVDLKMTSLTNEDTAMYFCATGNTYLDLW
GQGTLVTVSSTTTPAPRPPTPAPTIASQPLSLRPEACRPAAGGAVHTRGLDF

ACDIYIWAPLAGTCGVLLLSLVITLYCKRGRKKLLYIFKQPFMRPVQTTQE
EDGCSCRFPEEEEGGCELRVKFSRSADAPAYQQGQNQLYNELNLGRREEY
DVLDKRRGRDPEMGGKPRRKNPQEGLYNELQKDKMAEAYSEIGMKGER
RRGKGHDGLYQGLSTATKDTYDALHMQALPPR

SEQ ID NO: 54 PF0098 CAR

MALPVTALLLPLALLLHAARPDIVLTQSPASLAVSLGQRATISCRASES
VDYYGTSLMQWYQQKPGQPPKLLIYAASNVESGVPARFSGSGSGTDFSLN
IHPVEEDDIAMYFCQQTRKVYTFGGGTKLEIKGSTSGSGKPGSGEGSTKGQ
VQLQQPGAELVKPGASVKLSCKASGYTFTSYWMHWVKQRPGQGLEWIGE
IKPSNGLTNYIEKFKNKATLTVDKSATTAYMQLSSLTAEDSAVYYCTRTIT
TTEGYWFFDVWGAGTTVTVSSTTTPAPRPPTPAPTIASQPLSLRPEACRPAA
GGAVHTRGLDFACDIYIWAPLAGTCGVLLLSLVITLYCKRGRKKLLYIFKQ
PFMRPVQTTQEEDGCSCRFPEEEEGGCELRVKFSRSADAPAYQQGQNQLY
NELNLGRREEYDVLDKRRGRDPEMGGKPRRKNPQEGLYNELQKDKMAE
AYSEIGMKGERRRGKGHDGLYQGLSTATKDTYDALHMQALPPR

SEQ ID NO: 55 Rabbit IgG CH1-CH2-CH3

GQPKAPSVFPLAPCCGDTPSSTVTLGCLVKGYLPEPVTVTWNSGTLTN
GVRTFPSVRQSSGLYSLSSVVSVTSSSQPVTCNVAHPATNTKVDKTVAPST
CSKPTCPPPELLGGPSVFIFPPKPKDTLMISRTPEVTCVVVDVSQDDPEVQFT
WYINNEQVRTARPPLREQQFNSTIRVVSTLPIAHQDWLRGKEFKCKVHNK
ALPAPIEKTISKARGQPLEPKVYTMGPPREELSSRSVSLTCMINGFYPSDISV
EWEKNGKAEDNYKTTPAVLDSDGSYFLYSKLSVPTSEWQRGDVFTCSVM
HEALHNHYTQKSISRSPGK

SEQ ID NO: 56 Rabbit IgG CL

GDPVAPTVLIFPPAADQVATGTVTIVCVANKYFPDVTVTWEVDGTTQ
TTGIENSKTPQNSADCTYNLSSTLTSTQYNSHEYTCKVTQGTSVVQSFNRG
DC

SEQ ID NO: 57 22G5 VH-Rabbit IgG CH1-CH2-CH3

MGWSCIILFLVATATGVHSQSVEESGGRLVTPGTPLTLTCTVSGIDLSS
YAMTWVRQAPGEGLEWIGTINTISTAYANWAKGRFTISKTSTTVDLKIASP
TTEDTATYFCARDHGYYDVWGQGTLVTVSSGQPKAPSVFPLAPCCGDTPS
STVTLGCLVKGYLPEPVTVTWNSGTLTNGVRTFPSVRQSSGLYSLSSVVSV
TSSSQPVTCNVAHPANTKVDKTVAPSTCSKPTCPPPELLGGPSVFIFPPKPKP
KDTLMISRTPEVTCVVVDVSQDDPEVQFTWYINNEQVRTARPPLREQFNST

IRVVSTLPIAHQDWLRGKEFKCKVHNKALPAPIEKTISKARGQPLEPKVYT
MGPPREELSSRSVSLTCMINGFYPSDISVEWEKNGKAEDNYKTTPAVLDSD
GSYFLYSKLSVPTSEWQRGDVFTCSVMHEALHNHYTQKSISRSPGK

SEQ ID NO: 58 22G5 VL-Rabbit IgG CL

MGWSCIILFLVATATGVHSAQVLTQTPSSVSAAVGGTVTINCQASQSV
YKNNYLSWYQQKPGQPPRLLIYDASTLASGVPSRFKGSGSGTQFTLTISGV
QCDDAATYYCLGEFSCSSGDCAAFGGGTEVVVKGDPVAPTVLIFPPAADQ
VATGTVTIVCVANKYFPDVTVTWEVDGTTQTTGIENSKTPQNSADCTYNL
SSTLTLTSTQYNSHKEYTCKVTQGTTSVVQSFNRGDC

SEQ ID NO: 59 PF0098 VH-Rabbit IgG CH1-CH2-CH3

MGWSCIILFLVATATGVHSQVQLQPGAELVKPGASVKLSCKASGYTF
TSYWMHWVKQRPGQGLEWIGEIKPSNGLTNYIEKFKNKATLTVDKSATTA
YMQLSSLTAEDSAVYYCTRTITTTEGYWFFDVWGAGTTVTVSSGQPKAPS
VFPLAPCCGDTPSSTVTLGCLVKGYLPEPVTVTWNSGTLTNGVRTFPSVRQ
SSGLYSLSSVVSVTSSSQPVTCNVAHPATNTKVDKTVAPSTCSKPTCPPPEL
LGPSVFIFPPKPKPKDTLMISRTPEVTCVVVDVSQDDPEVQFTWYINNEQV
RTARPPLREQQFNSTIRVVSTLPIAHQDWLRGKEFKCKVHNKALPAPIEKTI
SKARGQPLEPKVYTMGPPREELSSRSVSLTCMINGFYPSDISVEWEKNGKA
EDNYKTTPAVLDSDGSYFLYSKLSVPTSEWQRGDVFTCSMHEALHNHYTQ
KSISRSPGK

SEQ ID NO: 60 PF0098 VL-Rabbit IgG CL

MGWSCIILFLVATATGVHSDIVLTQSPASLAVSLGQRATISCRASESVD
YYGTSLMQWYQQKPGQPPKLLIYAASNVESGVPARFSGSGTDFSLNIHPVE
EDDIAMYFCQQTRKVYTFGGGTKLEIKGDPVAPTVLIFPPAADQVATGTVT
IVCVANKYFPDVTVTWEVDGTTQTTGIENSKTPQNSADCTYNLSSTLTSTQ
YNSHKEYTCKVTQGTTSVVQSFNRGDC

SEQ ID NO: 61 CD8α signal peptide
MALPVTALLLPLALLLHAARP
SEQ ID NO: 62 CD8α hinge region
TTTPAPRPPTPAPTIASQPLSLRPEACRPAAGGAVHTRGLDFACD
SEQ ID NO: 63 CD8α transmembrane domain
IYIWAPLAGTCGVLLSLVITLYC
SEQ ID NO: 64 CD137 costimulatory signal domain
KRGRKLLYIFKQPFMRPVQTTQEEDGCSCRFPEEEEGGCEL
SEQ ID NO: 65 CD3ζ intracellular signalling domain

RVKFSRSADAPAYQGQNQLYNELNLGRREEYDVLDKRRGRDPEMGG
KPRRKNPQEGLYNELQKDKMAEAYSEIGMKGERRRGKGHDGLYQGLSTA
TKDTYDALHMQALPPR

SEQ ID NO: 66 Kozak sequence
gccgccacc
SEQ ID NO: 67 Linker 1
GGGGSGGGGSGGGGS
SEQ ID NO: 68 Linker 2
GSTSGSGKPGSGEGSTK
SEQ ID NO: 69 Linker repeat unit
GGGGS
SEQ ID NO: 70 Linker
GGGGSGGGGSGGGGSGGGGS
SEQ ID NO: 71 14C2 CAR-signal peptide-free

DVVMTQTPSSVSEPVGGTVTIKCQASQSISSYLAWYQQKPGQPPKLLI
YKASTLASGVSSQFRGSGSGTEFTLTISDLECADAATYYCQSTYAASNKLH
FAFGGGTDVVVKGGGGSGGGGSGGGGSQSLEESGGRLVTPGTPLTLTCTA
SGFSLSNYDMSWVRQAPGKGLEWIGTIEPDGSTWYASWAKGRLTISRTST
TVDLKMTSLTTEDTATYFCVRGAPDYSSNAFNLWGQGTLVTVSSTTTPAP
RPPTPAPTIASQPLSLRPEACRPAAGGAVHTRGLDFACDIYIWAPLAGTCGV
LLLSLVITLYCKRGRKKLLYIFKQPFMRPVQTTQEEDGCSCRFPEEEEGGCE
LRVKFSRSADAPAYQQGQNQLYNELNLGRREEYDVLDKRRGRDPEMGGK
PRRKNPQEGLYNELQKDKMAEAYSEIGMKGERRRGKGHDGLYQGLSTAT
KDTYDALHMQALPPR

SEQ ID NO: 72 21E1 CAR-signal peptide-free

AQVLTQTASPVSAAVGGTVTISCQSSQSVANNNLAWYQQKPGQPPSP LIYKASTLESGVPSRFSGSGSGTQFALTISGVQCDDAATYYCQGAYRSGIM AFGGGTEVVRGGGGSGGGGSGGGGSQSLEESGGRLVTPGTPLTLTCTVS GIDLSSYDMTWVRQAPGKGLEWIGSIGDDGNTYYATWAKGRFTISKTSTT VHLKMTSLTTEDTATYFCARGWSFCKSWGQGTLVTVSSTTTPAPRPPTPAP TIASQPLSLRPEACRPAAGGAVHTRGLDFACDIYIWAPLAGTCGVLLLSLVI TLYCKRGRKKLLYIFKQPFMRPVQTTQEEDGCSCRFPEEEEGGCELRVKFS RSADAPAYQQGQNQLYNELNLGRREEYDVLDKRRGRDPEMGGKPRRKNP

QEGLYNELQKDKMAEAYSEIGMKGERRRGKGHDGLYQGLSTATKDTYD ALHMQALPPR

SEQ ID NO: 73 22G5 CAR-signal peptide-free

AQVLTQTPSSVSAAVGGTVTINCQASQSVYKNNYLSWYQQKPGQPPR LLIYDASTLASGVPSRFKGSGSGTQFTLTISGVQCDDAATYYCLGEFSCSSG DCAAFGGGTEVVVKGGGGSGGGGSGGGGSQSVEESGGRLVTPGTPLTLTC TVSGIDLSSYAMTWVRQAPGEGLEWIGTINTISTAYYANWAKGRFTISKTS TTVDLKIASPTTEDTATYFCARDHGYYDVWGQGTLVTVSSTTTPAPRPPTP APTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRV KFSRSADAPAYQQGQNQLYNELNLGRREEYDVLDKRRGRDPEMGGKPRR KNPQEGLYNELQKDKMAEAYSEIGMKGERRRGKGHDGLYQGLSTATKDT YDALHMQALPPR

SEQ ID NO: 74 25E2 CAR-signal peptide-free

AYDMTQTPASVEVAVGGTVSIKCQVSQSIGSWLAWYQQKPGQPPKL
LIYSASTLASGVSSRFKGSGSGTEFTLTISDLACVDAATYYCQSYSSASTTA
FGGGTEVVVRGGGGSGGGGSGGGGSQSVEESGGRLVTPGTPLTLTCTVSG
IDLNKHAMGWVRQAPTKGLEWIGTIYVSTNTAYASWAKGRFTISKTSSTT
VDLKMTSLTTEDTATYFCARGGGSSYYTGELYFNLWGQGTLVTVSSTTTP
APRPPTPAPTIASQPLSLRPEACRPAAGGAVHTRGLDFACDIYIWAPLAGTC
GVLLLSLVITLYCKRGRKKLLYIFKQPFMRPVQTTQEEDGCSCRFPEEEEG
GCELRVKFSRSADAPAYQQGQNQLYNELNLGRREEYDVLDKRRGRDPEM
GGKPRRKNPQEGLYNELQKDKMAEAYSEIGMKGERRRGKGHDGLYQGLS
TATKDTYDALHMQALPPR

SEQ ID NO: 75 48D5 CAR-signal peptide-free

AQVLTQTPSSVSAAVGGTVTINCQSSQSVYGADYLAWYQQKPGQPPK
LLIYRASNLASGVPSRFRGSGSGTQFTLTISEVQCDDAATYYCQGTYISSDW
LNPFGGGTEVVVKGGGGSGGGGSGGGGSQSLEESGGRLVKPDETLTITCT
VSGIDLSSNAISWVRQAPGEGLEYIGYIGTGGKSFYATWTKGRFTISKTSTT
VDLKMTSLTNEDTAMYFCATGNTYLDLWGQGTLVTVSSTTTPAPRPPTPA
PTIASQPLSLRPEACRPAAGGAVHTRGLDFACDIYIWAPLAGTCGVLLLSL
VITLYCKRGRKKLLYIFKQPFMRPVQTTQEEDGCSCRFPEEEEGGCELRVK
FSRSADAPAYQQGQNQLYNELNLGRREEYDVLDKRRGRDPEMGGKPRRK

NPQEGLYNELQKDKMAEAYSEIGMKGERRRGKGHDGLYQGLSTATKDTY
DALHMQALPPR

**Detailed Description of Embodiments**

Embodiments of the present disclosure will be described in detail below with reference to examples; however, those skilled in the art will understand that the following examples are only used to illustrate the present disclosure and should not be regarded as limiting the scope of the present disclosure. For examples in which specific conditions are not specified, they are carried out according to the conventional conditions or the conditions recommended by the manufacturer. The reagents or instruments used without indicating the manufacturer are all commercially available conventional products.

**Example 1: Screening of anti-GCC rabbit monoclonal antibodies**

New Zealand rabbits were immunised with GCC protein via a service order from Genscript Biotech Corporation (Genscript, Cat. NO. C5825HF280), yielding antibodies with high affinity for the GCC protein. The specific screening method and steps are described below.

*1. Target cell construction*

The Dubca-human GCC.Luc and Dubca-mouse GCC.Luc overexpression cell lines were constructed using the following method. The coding sequences of human GCC (NM_004963.3 ) and mouse GCC (NM_001127318.1 ) were synthesised and separately inserted into a lentiviral expression vector pLVX-Luc-puro (Clontech, Cat. No. 632164 ). Subsequently, each of the resulting vectors was co-transfected with helper packaging plasmids psPAX2 and pMD.2G into Lenti-X 293T host cells, and lentiviruses were obtained via concentration and purification. The two lentiviruses obtained were separately transduced into the Dubca cell line (ATCC® CRL2276™). After 3 rounds of puromycin resistance screening, the Dubca-human GCC.Luc and Dubca-mouse GCC.Luc were obtained.

The expression level of the human GCC target was detected by incubating the Dubca-human GCC.Luc cells with the 5F9-hIgGFc antibody (Genscript, Cat. NO. C0224DK260-1). Dubca-human GCC.Luc cells and Dubca cells ($2.0 \times 10^5$ each) were incubated with the 5F9-hIgGFc antibody diluted 1 : 100 for 0.5 hours at 4°C; the cells were washed three times with DPBS, then resuspended in a fluorochrome-conjugated secondary antibody (PE anti-human IgG Fc Antibody, Biolegend, Cat. No. 409304) diluted 1 : 100, and incubated for 0.5 hours at 4°C in the dark; after washing three times with DPBS, the cells were loaded onto a flow cytometer and the data were read. The expression level of the mouse GCC target was detected by incubating the Dubca-mouse GCC.Luc cells with the expressed and purified PF0098-Rabbit IgG antibody, using the same detection method as described above. PF0098 is an anti-GCC antibody disclosed in patent application WO 2019224716 A2 (VH set forth in SEQ ID NO: 36 and VL set forth in SEQ ID NO: 42), which can recognise human GCC, monkey GCC, and mouse GCC simultaneously, and can be used for immunohistochemical detection. This antibody will also be cited as a standard control in subsequent examples.

Using the method described above, CT26 cell line (ATCC, CRL-2638TM and HEK293T cell line (Clontech, Cat. No. 632180) were made to overexpress mouse GCC (NM_001127318.1) or human GCC (NM_004963.3) to construct CT26-mouse GCC. Luc, 293T-mouse GCC. Luc, and 293T-human GCC. Luc cells. HEK293T cell line (Clontech, Cat. No. 632180) and colon cancer cell line LS1034 (ATCC, CRL-2158TM), which natively expresses human GCC at high levels, were also constructed to express firefly luciferase, yielding LS1034.Luc cells, which were used for subsequent in vitro screening.

## 2. Animal immunisation and titre detection

Anti-GCC rabbit monoclonal antibodies were produced via a service order using the MonoRab™ platform from Genscript Biotech Corporation. Two New Zealand white rabbits were immunised by a conventional immunisation method using His-tagged mouse GUCY2C/guanylyl cyclase C protein (ACRO GUC-M52H3, abbreviated as mouse GCC-his protein), which had been modified with the ImmunoPlus™ technology, with an immunisation interval of two weeks; to enhance the immune effect, 293T-mouse GCC cells were used for the third round of immunisation. On day 7 after the end of the four rounds of immunisation, blood was collected and titre was detected against two target proteins, mouse GCC-his and His-tagged human GUCY2C/Guanylyl cyclase C protein (ACRO GUC-H52H5, abbreviated as human GCC-his protein).

## 3. Positive clone screening

After completion of immunisation, 30 mL of fresh blood was collected from the rabbit for PBMC isolation. Through selection of antigen-positive B cell clones, the antigen-positive B cells were plated into 50 pieces of 96-well cell culture plates for culture. The binding characteristics of the cell supernatants to mouse GCC-his and human GCC-his target proteins were detected using indirect ELISA. The supernatants from clones showing specific cross-binding were selected for FACS binding validation against Dubca-mouse GCC.Luc cells and Dubca cells. The positive clones were selected and expanded into 24-well plates for continued culture. The supernatants after expansion culture were re-tested using the above ELISA and FACS methods for confirmation. The FACS detection method was as follows: Target cells were digested, collected, and aliquoted into 96-well V-bottom plates at $2 \times 10^5$ cells per well. After centrifugation at 400 g for 3 minutes, the supernatant was discarded. Then, 100 μL of the B cell clone supernatant was added, followed by co-incubation at 4°C for 1 hour. The cells were washed three times with DPBS buffer pre-chilled at 4°C. Alexa Fluor® 488 goat anti-rabbit IgG (H+L) (Invitrogen, Cat. NO. A11034) diluted 1 : 100 was added, followed by incubation at 4°C for 30 minutes, and the resulting mixture was then washed three times with DPBS pre-chilled at 4°C. The above incubated cells were loaded on an Attune NxT flow cytometer (Thermofisher) and the data were read. The results are shown in Table 2. The clone 14C2 bound only to the target cell Dubca-mouse GCC.Luc, while the remaining four clones were capable of specifically binding to the two types of target cells, namely Dubca-mouse GCC.Luc and Dubca-human GCC.Luc. Therefore, the four antibody clones obtained through screening exhibited cross-species reactivity with mouse and human GCC proteins.

**Table 2. Results of binding between B cell clone supernatants and target cells**

| B cell clone number | Dubca | Dubca-mouse GCC.Luc | Dubca-human GCC.Luc |
|---|---|---|---|
| 14C2-B cells | 3.07% | 99.16% | 5.13% |

(continued)

| B cell clone number | Dubca | Dubca-mouse GCC.Luc | Dubca-human GCC.Luc |
|---|---|---|---|
| 21E1-B cells | 2.67% | 99.70% | 95.40% |
| 22G5-B cells | 2.16% | 74.13% | 99.86% |
| 25E2-B cells | 2.91% | 95.42% | 95.11% |
| 48D5-B cells | 4.30% | 81.84% | 65.94% |

Positive B cell clones were isolated using the MonoRab™ platform. RNA was extracted from the selected and confirmed positive B cell clones using TRIzol® reagent (Ambion, Cat. No.: 15596-026) according to the standard technical protocol. cDNA reverse transcription was performed using the PrimeScript™ 1st Strand cDNA Synthesis Kit (Takara, Cat. No.: 6110A) according to the instruction manual, followed by the amplification of light chain and heavy chain fragments of the antibody. The light chain and heavy chain sequences of the antibody were obtained through next-generation sequencing. Tables 3 and 4 show the antibody heavy chain (VH) and light chain (VL) variable region sequences, respectively, partitioned according to the Kabat numbering rules, along with the corresponding complementarity determining region (CDR) sequences. The heavy chain variable region (VH) and the light chain variable region (VL) of the antibody are linked by a linker that may consist of repeating GGGGS (SEQ ID NO: 69) amino acid sequences or variants thereof (e.g., SEQ ID NOs: 67-68).

**Table 3. Antibody heavy chain variable region and CDR amino acid sequences**

| Antibody number | VH | VH CDR1 | VH CDR2 | VH CDR3 |
|---|---|---|---|---|
| 14C2 | SEQ ID NO: 31 | NYDMS (SEQ ID NO: 1) | TIEPDGSTWYAS WAKG (SEQ ID NO: 6) | GAPDYSSNAF NL (SEQ ID NO: 11) |
| 21E1 | SEQ ID NO: 32 | SYDMT (SEQ ID NO: 2) | SIGDDGNTYYA TWAKG (SEQ ID NO: 7) | GWSFCKS (SEQ ID NO: 12) |
| 22G5 | SEQ ID NO: 33 | SYAMT (SEQ ID NO: 3) | TINTISTAYYAN WAKG (SEQ ID NO: 8) | DHGYYDV (SEQ ID NO: 13) |
| 25E2 | SEQ ID NO: 34 | KHAMG (SEQ ID NO: 4) | TIYVSTNTAYAS WAKG (SEQ ID NO: 9) | GGSSYYTGEL YFNL (SEQ ID NO: 14) |
| 48D5 | SEQ ID NO: 35 | SNAIS (SEQ ID NO: 5) | YIGTGGKSFYAT WTKG (SEQ ID NO: 10) | GNTYLDL (SEQ ID NO: 15) |

**Table 4. Antibody light chain variable region and CDR amino acid sequences**

| Antibody number | VL | VL CDR1 | VL CDR2 | VL CDR3 |
|---|---|---|---|---|
| 14C2 | SEQ ID NO: 37 | QASQSISSYLA (SEQ ID NO: 16) | KASTLAS (SEQ ID NO: 21) | QSTYAASNKLHFA (SEQ ID NO: 26) |
| 21E1 | SEQ ID NO: 38 | QSSQSVANNNLA (SEQ ID NO: 17) | KASTLES (SEQ ID NO: 22) | QGAYRSGIMA (SEQ ID NO: 27) |

(continued)

| Antibody number | VL | VL CDR1 | VL CDR2 | VL CDR3 |
|---|---|---|---|---|
| 22G5 | SEQ ID NO: 39 | QASQSVYKNNYLS (SEQ ID NO: 18) | DASTLAS (SEQ ID NO: 23) | LGEFSCSSGDCAA (SEQ ID NO: 28) |
| 25E2 | SEQ ID NO: 40 | QVSQSIGSWLA (SEQ ID NO: 19) | SASTLAS (SEQ ID NO: 24) | QSYSSASTTA (SEQ ID NO: 29) |
| 48D5 | SEQ ID NO: 41 | QSSQSVYGADYLA (SEQ ID NO: 20) | RASNLAS (SEQ ID NO: 25) | QGTYISSDWLNP (SEQ ID NO: 30) |

## Example 2: Preparation. purification and identification of candidate antibodies

Rabbit IgG molecules are categorised into IgG-k and IgG-$\lambda$ depending on the light chain. In general, antibodies obtained from New Zealand white rabbits are mostly IgG-k, with only < 1% being IgG-$\lambda$. The native rabbit IgG-k has a molecular weight of about 150 KD and comprises two identical light chains and two identical heavy chains. Each of the light chains consists of an N-terminal variable domain VL and a constant domain CL (e.g., SEQ ID NO: 56), and each of the heavy chains consists of an N-terminal variable domain VH and three constant domains (CH1, CH2, and CH3, e.g., SEQ ID NO: 55), wherein the VL and VH each contain three complementarity determining regions CDR1, CDR2, and CDR3. The light and heavy chains, as well as the two heavy chains, are linked via disulphide bonds.

The above screened heavy chain variable region and light chain variable region sequences were separately fused to rabbit IgG (IgG1k), e.g., 22G5 single chain variable fragment sequence or PF0098 single chain variable fragment sequence to obtain 22G5 VH-Rabbit IgG CH1-CH2-CH3 (SEQ ID NO: 57) and 22G5 VL-Rabbit IgG CL (SEQ ID NO 58) or PF0098 VH-Rabbit IgG CH1-CH2-CH3 (SEQ ID NO: 59) and PF0098 VL-Rabbit IgG CL (SEQ ID NO: 60), respectively. The coding sequences of the fusion sequences were separately inserted into the pcDNA3.4 vector to construct expression plasmids. Subsequently, the 22G5-Rabbit IgG antibody and the PF0098-Rabbit IgG antibody were expressed in an HD CHO-S cell culture and further purified. The concentration of the obtained antibody was determined using Nano Drop A280nm, and the purity was evaluated by means of SDS-PAGE. Table 5 shows the purification information of antibodies 22G5-Rabbit IgG and PF0098-Rabbit IgG. This indicates that the 22G5 antibody can be recombinantly expressed with high efficiency.

### Table 5. Antibody purification information

| Antibody name | Expression system | Concentration mg/mL | SDS-PAGE purity% | Total yield mg |
|---|---|---|---|---|
| 22G5-Rabbit IgG | 20 mL | 0.6 | > 99 | 7.88 |
| PF0098-Rabbit IgG | 300 mL | 1.69 | > 85 | 8.45 |

The binding capacity of the purified antibodies to the target proteins was detected using an enzyme-linked immunosorbent assay. The two target proteins, human GCC-his and mouse GCC-his, were separately diluted in a coating solution to a concentration of 1 $\mu$g/mL. Each well of a 96-well Costar plate was coated with 100 $\mu$L of the diluted protein solution and incubated overnight at 4°C. The next day, after washing twice with a 0.05% PBST washing solution, blocking was performed with 5% MPBS at room temperature for 2 hours; after completion of blocking, the plates were washed and then incubated with serially diluted antibody solutions at room temperature for 1.5 hours; after completion of incubation, the plates were washed three times with washing solution, followed by the addition of an enzyme-conjugated secondary antibody (mouse anti-rabbit IgG antibody (M205) [HRP], mAb (Genscript, Cat# A01827)) diluted 1 : 10000. The plates were incubated at room temperature for 45 minutes. The plates were washed four times with a washing solution, followed by the addition of TMB for colour development for 10 minutes. Subsequent, the colour development reaction was terminated by adding 1 M HCl. The absorbance at OD450 nm was then measured using a microplate reader, and the data were analysed. Binding curves are shown in FIG. 1A and FIG. 1B, and the $EC_{50}$ is shown in Table 6. The results show that both the 22G5-Rabbit IgG antibody and the PF0098-Rabbit IgG antibody have a strong binding capacity to the GCC protein.

### Table 6. Antibody-target protein binding $EC_{50}$

| $EC_{50}$ | 22G5-Rabbit IgG | PF0098-Rabbit IgG | Rabbit IgG |
|---|---|---|---|
| Human GCC-his protein | 0.6194 nM | 0.6707 nM | NA |

(continued)

| EC$_{50}$ | 22G5-Rabbit IgG | PF0098-Rabbit IgG | Rabbit IgG |
|---|---|---|---|
| Mouse GCC-his protein | 0.8236 nM | 0.8993 nM | NA |

The binding capacity of the purified antibodies to the target cells was detected by flow cytometry. 293T-human GCC.Luc and 293T-mouse GCC.Luc cells were used as target cells, with 293T cells serving as the negative control. The cells were seeded into a 96-well plate at a density of 0.2 M per well, followed by incubation with serially diluted antibodies at 4°C for 60 minutes; subsequently, the cells were washed three times with DPBS, and the supernatants were discarded. The cell pellet was resuspended in Alexa Fluor® 488 goat anti-rabbit IgG (H+L) (Invitrogen, Cat. NO. A11034) diluted 1 : 100, followed by incubation at 4°C in the dark for 30 minutes. After washing three times with DPBS, the cells were loaded onto a flow cytometer and the data were read. As shown in FIG. 2A to FIG. 2C, the binding of both the 22G5-Rabbit IgG antibody and the PF0098-Rabbit IgG antibody to target cells was strongly concentration-dependent. The EC$_{50}$ is shown in Table 7. Furthermore, the 22G5-Rabbit IgG showed no binding to 293T cells, whereas the PF0098-Rabbit IgG antibody showed strong non-specific binding to 293T cells at high concentrations. In summary, the 22G5-Rabbit IgG has a higher specificity for the GCC antigen.

**Table 7. Antibody-target cell binding EC$_{50}$**

| EC$_{50}$ | 22G5-Rabbit IgG | PF0098-Rabbit IgG | Rabbit IgG |
|---|---|---|---|
| 293T-human GCC.Luc | 0.1021 nM | 0.02541 nM | NA |
| 293T-mouse GCC.Luc | 1.228 nM | 0.2843 nM | NA |

### Example 3: Immunohistochemical detection of candidate antibodies

Paraffin-embedded tissue sections for detection were subjected to deparaffinisation, followed by sequential immersion in 100% ethanol, 95% ethanol, 80% ethanol, 70% ethanol and pure water. Subsequently, antigen retrieval was performed on the sections by microwave heating using a modified sodium citrate antigen retrieval solution (BEYOTIME #P0083). After the antigen retrieval system was cooled down, the sections were washed with PBS, followed by sequential blocking treatment with 10% hydrogen peroxide (Sinopharm Chemical Reagent Co., Ltd. #10011218) and 5% goat serum (BEYOTIME #C0265). The anti-GCC antibody was diluted to a working concentration of 0.5 μg/mL using an antibody diluent (Beijing Zhongshan Golden Bridge Biotechnology Co., Ltd. #ZLI-9030). The goat serum on the sections was removed, and the diluted antibody was added dropwise onto the tissue sections, followed by incubation overnight at 4°C. After completion of antibody incubation, the sections were washed three times with PBS. The goat anti-rabbit antibody from a ready-to-use rapid immunohistochemistry detection kit (Fuzhou Maixin Biotechnology Development Co., Ltd. #KIT-5005) was added dropwise onto the tissue sections, followed by incubation at room temperature in the dark for 15 minutes. After completion of antibody incubation, the sections were washed three times with PBS, followed by colour development using a DAB developer (VECTOR LABORATORIES#sk-4105). After completion of colour development, the sections were stained with haematoxylin staining solution (BaSO Biotechnology Co., Ltd. #BA4097). Subsequently, the sections were subjected to sequential immersion in 70% ethanol, 80% ethanol, 95% ethanol, 100% ethanol, and xylene. After completion of the treatment, neutral resin (Sinopharm Chemical Reagent Co., Ltd. #10004160) was added dropwise onto the tissue sections, followed by mounting. After the sections were dried, observation and photography were performed under a microscope.

The experimental results are shown in FIG. 3. The isotype control antibody ab172730 (Abcam, recombinant rabbit IgG, monoclonal [EPR25A]-isotype control) showed no staining in the four types of tissue sections. The PF0098-Rabbit IgG antibody exhibited strong GCC-specific staining in the sections of SW948 subcutaneous tumour and LS1034 subcutaneous tumour, and produced non-specific staining in mouse spleen. Under the same experimental conditions and at an antibody concentration of 0.5 μg/mL, the 22G5-Rabbit IgG antibody exhibited strong specific staining in the sections of GCC-expressing SW948 subcutaneous tumour, LS1034 subcutaneous tumour, and mouse small intestine tissue; the membrane staining intensity was higher than that of the PF0098-Rabbit IgG antibody. Furthermore, no non-specific staining was observed in the section of mouse spleen tissue without GCC expression. In addition, the anti-GCC antibody ab122404 (Abcam) exhibited cytoplasm-localised staining in the sections of SW948 subcutaneous tumour and LS1034 subcutaneous tumour, showed no specific staining on the cell membrane surface, and produced non-specific staining in the mouse small intestine and the mouse spleen. Therefore, the 22G5-Rabbit IgG antibody exhibits superior cell membrane staining intensity and specificity for GCC-positive cells compared with the control antibody. The 22G5-Rabbit IgG antibody is a novel anti-GCC antibody suitable for immunohistochemical detection of human and mouse tissue sections.

**Example 4: Construction of CAR-T cells**

A nucleotide molecule encoding the backbone structural sequence of a chimeric antigen receptor (CAR) was synthesised using gene synthesis technology. The encoded structures from the N-terminus to the C-terminus were respectively as follows: CD8α hinge region (SEQ ID NO: 62), CD8α transmembrane domain (SEQ ID NO: 63), CD137 costimulatory signalling domain (SEQ ID NO: 64), and CD3ζ intracellular signalling domain (SEQ ID NO: 65). This synthesised nucleotide molecule was cloned into a lentiviral vector (pLSINK-BBzBB), and the resulting backbone vector was designated as "pLSINK-CE11A16". The multiple cloning site (MCS) within the vector enabled the nucleic acid sequence containing Kozak sequence (SEQ ID NO: 66) to be operably linked to the CD8α signal peptide (SEQ ID NO: 61) of the CAR backbone sequence and subsequently inserted into the upstream of the CAR backbone vector. Using molecular cloning techniques known in the art, the synthesised nucleic acid sequence encoding the CD8α signal peptide and the anti-GCC single chain variable fragment (e.g., 22G5 scFv, SEQ ID NO: 45) was cloned into the lentiviral vector pLSINK-CE11A16 via the EcoRI (5'-GAATTC-3') and SpeI (5'-ACTAGT-3') restriction sites. The constructed chimeric antigen receptors (CARs) were 14C2 CAR, 21E1 CAR, 22G5 CAR, 25E2 CAR, 48D5 CAR, and PF0098 CAR, respectively (e.g., SEQ ID NOs: 49-53 and SEQ ID NO: 54).

A mixture of lentiviral helper packaging plasmids containing pMDLg.pRRE (Addgene #12251), pRSV-REV (Addgene #12253), and pMD2.G (Addgene #12259) was mixed with the vector expressing the CAR construct at a pre-optimised ratio. The resulting mixture was then mixed with polyetherimide (PEI), followed by incubation at 25°C for 5 minutes. The mixture was then added to HEK293 cells for transfection. Subsequently, the cells were incubated overnight in a cell culture incubator at 37°C with 5% $CO_2$. The supernatant was collected and centrifuged at 3000 g for 15 minutes at 4°C. Pre-formulated 20% PEG 8000 was added to the supernatant at 4 : 1, and mixed uniformly by gentle inversion, followed by overnight precipitation of the virus at 4°C. Then centrifugation was performed at 3000g for 30 minutes at 4°C, the supernatant was carefully discarded, and the virus pellet was carefully resuspended in pre-chilled DPBS and stored at -80°C for later use. The viral titre was determined by transduction titration using the SUP-T1 (human T lymphoblast) cell line.

Human T cells were purified from commercial PBMCs using a Miltenyi Pan T cell isolation kit (Cat# 130-096-535) according to the manufacturer's protocol as follows. The cell count was first determined, and the cell suspension was centrifuged at 300 g for 10 minutes. Then the supernatant was completely discarded, and the cell pellet was resuspended in 40 μL of MACS buffer (DPBS supplemented with 8 μM EDTA and 0.5% FBS) per $10^7$ total cells. For every $10^7$ total cells, 10 μL of Pan T Cell Biotin-Antibody Cocktail was added, mixed uniformly, and incubated in a refrigerator (2°C to 8°C) for approximately 5 minutes. Then 30 μL of MACS buffer was added per $10^7$ cells. For every $10^7$ total cells, 20 μL of Pan T Cell MicroBead Cocktail was added. The cell suspension mixture was mixed thoroughly and incubated in a refrigerator (2°C to 8°C) for another 10 minutes. At least 500 μL of the system was required for magnetic separation. For magnetic separation, an LS column was placed in the magnetic field of a suitable MACS separator. The column was pretreated by rinsing with 3 mL of buffer. The cell suspension was then loaded onto the column, and the flow-through containing unlabelled cells was collected, wherein the flow-through represented the enriched T cell fraction. The remaining T cells were collected by washing the column with 3 mL of buffer and collecting the passed unlabelled cells. The two collected T cell fractions were combined, centrifuged, and resuspended in TexMACS GMP Medium&1L (Miltenyi, Cat#170-076-309) containing 300 IU/mL IL-2.

The prepared T cells were then activated for 48 hours using human T Cell TransAct™ (Miltenyi #130-111-160), wherein the ratio of anti-CD3/CD28 MACSiBead beads added to the T cells was 1 : 2.

The activated T cells were transduced with the lentivirus at a certain multiplicity of infection; 72 hours later, an R10 medium containing 300 IU/mL IL-2 was supplemented, and the transduced cells were transferred to a new cell culture plate for cell expansion under the conditions of 37°C and 5% $CO_2$.

The CAR expression levels were assessed by flow cytometry using directly labelled antibodies. On day 6 post-transduction, $5 \times 10^5$ T cells were collected from each group. The antibodies were prepared using pre-chilled DPBS buffer. PF0098 CAR-T cells and non-transduced CAR T cells (UnT) were incubated with Alexa Fluor® 488 goat anti-mouse IgG (H+L) (Invitrogen, Cat. NO. A11001) diluted 1 : 100 for 30 minutes at 4°C. The remaining CAR-T cells and the non-transduced UnT cells were incubated with Alexa Fluor® 488 goat anti-rabbit IgG (H+L) (Invitrogen, Cat. NO. A11034) diluted 1 : 100 for 30 minutes at 4°C. Following incubation, all cells were washed three times with pre-chilled DPBS at 4°C. The above incubated cells were loaded on an Attune NxT flow cytometer (Thermofisher) and the data were read. The results are shown in Table 8, showing CAR expressions ranging from 43.94% to 75.10%, with PF0098 CAR-T serving as the control and UnT representing non-transduced CAR T cells.

**Table 8. CAR expression levels in T cells post-transduction**

| CAR-T number | CAR positivity rate (%) |
|---|---|
| UnT | 1.22%/0.08% |

(continued)

| CAR-T number | CAR positivity rate (%) |
|---|---|
| PF0098 CAR-T | 43.94% |
| 14C2 CAR-T | 65.70% |
| 21E1 CAR-T | 62.18% |
| 22G5 CAR-T | 68.73% |
| 25E2 CAR-T | 58.05% |
| 48D5 CAR-T | 75.10% |

### Example 5: Functional assessment of CAR-T cells

The CAR-T cells prepared in Example 4 were subjected to cytotoxicity assay. The above CAR-T cells were co-incubated with the LS1034.Luc, CT26-mouse GCC.Luc and 293T.Luc cells prepared in Example 1 at effector-to-target ratios (E/T) of 5 : 1, 2.5 : 1, and 1.25 : 1 for 24 hours, respectively. PF0098 CAR-T served as the positive control. To determine the cytotoxicity of CAR-T cells against target cells, a One-glo luciferase assay reagent (Promega #E6120) was added to the co-cultured cells to detect the remaining luciferase activity in the well. The remaining luciferase activity is directly related to the number of remaining target cells in the well. Cytotoxicity was calculated using the following formula:

$$\text{Cytotoxicity\%} = 100\% \times (1-(\text{RLU}_{sample}-\text{RLU}_{min})/(\text{RLU}_{unT}-\text{RLU}_{min})).$$

$\text{RLU}_{sample}$ refers to the luciferase activity value measured in a well containing CAR-T cells transduced with anti-GCC CAR. $\text{RLU}_{min}$ refers to the luciferase activity value determined in a well where Triton X-100 was added at a final concentration of 1% at the beginning of the cytotoxicity assay. $\text{RLU}_{unT}$ refers to the luciferase activity value determined in a well containing non-transduced CAR T cells.

As shown in FIG. 4A to FIG. 4C, all CAR-T cells showed strong cytotoxicity against CT26-mouse GCC.Luc cells. As shown in FIG. 5A to FIG. 5C, 14C2 CAR-T cells exhibited no cytotoxicity effect on LS1034.Luc cells (natively expressing human GCC). This is because 14C2 does not bind to the human GCC protein. 25E2 CAR-T cells exhibited cytotoxicity against target cells only at a high E/T ratio of 5 : 1. The remaining three CAR-T cells all maintained strong cytotoxicity against the target cell LS1034.Luc. As shown in FIG. 6A to FIG. 6C, these CAR-T cells still exhibited no cytotoxicity against 293T.Luc even at higher E/T ratios, demonstrating that these five CAR-T cell types specifically recognise only GCC-positive target cells.

In addition to cytotoxicity against target cells, the ability of GCC CAR-T cells (prepared in Example 4) to produce IFN-$\gamma$ after co-incubation with target cells (LS1034.Luc, CT26-mouse GCC.Luc, and 293T.Luc cells prepared in Example 1) was also analysed. GCC CAR-T cells were mixed with target cells at an E/T ratio of 2.5 : 1 (2000 target cells per well) and co-incubated in a 384-well plate for 24 hours. The concentration of IFN-$\gamma$ produced in the culture supernatant after co-incubation of CAR-T with the target cells, as well as in the supernatant of CAR-T cells alone, was measured using an HTRF kit (Cisbio , Cat#62HIFNGPEG). Prior to the assay, the HTRF reagent required stabilisation at room temperature for 30 minutes prior to the assay. The co-culture supernatant was transferred to a 384-well assay plate (Greiner Bio-One, #784075 ) at 16 $\mu$L/well, followed by the addition of a pre-mixed HTRF reagent (prepared according to the kit operating manual) at 4 $\mu$L/well. The plate was sealed with a sealing film and incubated overnight at room temperature. HTRF values were read on a Tecan Spark 10M. The IFN-$\gamma$ concentrations were calculated from the signals obtained with reference to the standard curve provided by the kit.

As shown in FIG. 7A to FIG. 7D, the detected concentrations of IFN$\gamma$ secreted by the five CAR-T cell types ranged from 1488.3 pg/mL to 2692.5 pg/mL (co-incubated with CT26-mGCC.Luc target cells) and from 1173.1 pg/mL to 1939.0 pg/mL (co-incubated with LS1034.Luc target cells, except for 14C2 CAR-T), showing an upregulation relative to the UnT control (the detected values were 600.4 pg/mL and 910.8 pg/mL, respectively).

Although the specific embodiments of the present disclosure have been described in detail, it will be understood by a person skilled in the art that: according to all the teachings disclosed, various modifications and replacements can be made to those details, and these changes are within the scope of the protection of the present disclosure. The full scope of the present disclosure is given by the appended claims and any equivalents thereof.

**Claims**

1. An anti-GCC antibody or an antigen-binding fragment thereof, **characterized by** comprising a heavy chain variable region and a light chain variable region, the heavy chain variable region comprising heavy chain complementarity determining regions HCDR1 to HCDR3, and the light chain variable region comprising light chain complementarity determining regions LCDR1 to LCDR3, wherein

(1) the heavy chain variable region comprises HCDR1, HCDR2, and HCDR3 in an amino acid sequence set forth in SEQ ID NO: 31, and the light chain variable region comprises LCDR1, LCDR2, and LCDR3 in an amino acid sequence set forth in SEQ ID NO: 37;
(2) the heavy chain variable region comprises HCDR1, HCDR2, and HCDR3 in an amino acid sequence set forth in SEQ ID NO: 32, and the light chain variable region comprises LCDR1, LCDR2, and LCDR3 in an amino acid sequence set forth in SEQ ID NO: 38;
(3) the heavy chain variable region comprises HCDR1, HCDR2, and HCDR3 in an amino acid sequence set forth in SEQ ID NO: 33, and the light chain variable region comprises LCDR1, LCDR2, and LCDR3 in an amino acid sequence set forth in SEQ ID NO: 39;
(4) the heavy chain variable region comprises HCDR1, HCDR2, and HCDR3 in an amino acid sequence set forth in SEQ ID NO: 34, and the light chain variable region comprises LCDR1, LCDR2, and LCDR3 in an amino acid sequence set forth in SEQ ID NO: 40; or
(5) the heavy chain variable region comprises HCDR1, HCDR2, and HCDR3 in an amino acid sequence set forth in SEQ ID NO: 35, and the light chain variable region comprises LCDR1, LCDR2, and LCDR3 in an amino acid sequence set forth in SEQ ID NO: 41.

2. The anti-GCC antibody or the antigen-binding fragment thereof according to claim 1, **characterized in that** the HCDR1 to HCDR3 of the heavy chain variable region and the LCDR1 to LCDR3 of the light chain variable region are determined according to the Kabat numbering system, the AbM numbering system, the IMGT numbering system, the Chothia numbering system, or the contact numbering system.

3. An anti-GCC antibody or an antigen-binding fragment thereof, **characterized by** comprising a heavy chain variable region and a light chain variable region, the heavy chain variable region comprising heavy chain complementarity determining regions HCDR1 to HCDR3, and the light chain variable region comprising light chain complementarity determining regions LCDR1 to LCDR3, wherein

(1) the amino acid sequence of the HCDR1 is set forth in SEQ ID NO: 1, the amino acid sequence of the HCDR2 is set forth in SEQ ID NO: 6, the amino acid sequence of the HCDR3 is set forth in SEQ ID NO: 11, the amino acid sequence of the LCDR1 is set forth in SEQ ID NO: 16, the amino acid sequence of the LCDR2 is set forth in SEQ ID NO: 21, and the amino acid sequence of the LCDR3 is set forth in SEQ ID NO: 26;
(2) the amino acid sequence of the HCDR1 is set forth in SEQ ID NO: 2, the amino acid sequence of the HCDR2 is set forth in SEQ ID NO: 7, the amino acid sequence of the HCDR3 is set forth in SEQ ID NO: 12, the amino acid sequence of the LCDR1 is set forth in SEQ ID NO: 17, the amino acid sequence of the LCDR2 is set forth in SEQ ID NO: 22, and the amino acid sequence of the LCDR3 is set forth in SEQ ID NO: 27;
(3) the amino acid sequence of the HCDR1 is set forth in SEQ ID NO: 3, the amino acid sequence of the HCDR2 is set forth in SEQ ID NO: 8, the amino acid sequence of the HCDR3 is set forth in SEQ ID NO: 13, the amino acid sequence of the LCDR1 is set forth in SEQ ID NO: 18, the amino acid sequence of the LCDR2 is set forth in SEQ ID NO: 23, and the amino acid sequence of the LCDR3 is set forth in SEQ ID NO: 28;
(4) the amino acid sequence of the HCDR1 is set forth in SEQ ID NO: 4, the amino acid sequence of the HCDR2 is set forth in SEQ ID NO: 9, the amino acid sequence of the HCDR3 is set forth in SEQ ID NO: 14, the amino acid sequence of the LCDR1 is set forth in SEQ ID NO: 19, the amino acid sequence of the LCDR2 is set forth in SEQ ID NO: 24, and the amino acid sequence of the LCDR3 is set forth in SEQ ID NO: 29; or
(5) the amino acid sequence of the HCDR1 is set forth in SEQ ID NO: 5, the amino acid sequence of the HCDR2 is set forth in SEQ ID NO: 10, the amino acid sequence of the HCDR3 is set forth in SEQ ID NO: 15, the amino acid sequence of the LCDR1 is set forth in SEQ ID NO: 20, the amino acid sequence of the LCDR2 is set forth in SEQ ID NO: 25, and the amino acid sequence of the LCDR3 is set forth in SEQ ID NO: 30.

4. The anti-GCC antibody or the antigen-binding fragment thereof according to any one of claims 1 to 3, **characterized in that**

(1) the heavy chain variable region comprises an amino acid sequence having at least 80% identity to the amino acid sequence set forth in SEQ ID NO: 31, and the light chain variable region comprises an amino acid sequence having at least 80% identity to the amino acid sequence set forth in SEQ ID NO: 37;

(2) the heavy chain variable region comprises an amino acid sequence having at least 80% identity to the amino acid sequence set forth in SEQ ID NO: 32, and the light chain variable region comprises an amino acid sequence having at least 80% identity to the amino acid sequence set forth in SEQ ID NO: 38;

(3) the heavy chain variable region comprises an amino acid sequence having at least 80% identity to the amino acid sequence set forth in SEQ ID NO: 33, and the light chain variable region comprises an amino acid sequence having at least 80% identity to the amino acid sequence set forth in SEQ ID NO: 39;

(4) the heavy chain variable region comprises an amino acid sequence having at least 80% identity to the amino acid sequence set forth in SEQ ID NO: 34, and the light chain variable region comprises an amino acid sequence having at least 80% identity to the amino acid sequence set forth in SEQ ID NO: 40; or

(5) the heavy chain variable region comprises an amino acid sequence having at least 80% identity to the amino acid sequence set forth in SEQ ID NO: 35, and the light chain variable region comprises an amino acid sequence having at least 80% identity to the amino acid sequence set forth in SEQ ID NO: 41.

5. The anti-GCC antibody or the antigen-binding fragment thereof according to any one of claims 1 to 4, **characterized in that**

(1) the amino acid sequence of the heavy chain variable region is set forth in SEQ ID NO: 31, and the amino acid sequence of the light chain variable region is set forth in SEQ ID NO: 37;

(2) the amino acid sequence of the heavy chain variable region is set forth in SEQ ID NO: 32, and the amino acid sequence of the light chain variable region is set forth in SEQ ID NO: 38;

(3) the amino acid sequence of the heavy chain variable region is set forth in SEQ ID NO: 33, and the amino acid sequence of the light chain variable region is set forth in SEQ ID NO: 39;

(4) the amino acid sequence of the heavy chain variable region is set forth in SEQ ID NO: 34, and the amino acid sequence of the light chain variable region is set forth in SEQ ID NO: 40; or

(5) the amino acid sequence of the heavy chain variable region is set forth in SEQ ID NO: 35, and the amino acid sequence of the light chain variable region is set forth in SEQ ID NO: 41.

6. The anti-GCC antibody or the antigen-binding fragment thereof according to any one of claims 1 to 5, **characterized in that** the anti-GCC antibody comprises a non-CDR region, and the non-CDR region is from a human, a mouse, or a rabbit;

preferably, the non-CDR region comprises a framework region, a heavy chain constant region, and/or a light chain constant region.

7. The anti-GCC antibody or the antigen-binding fragment thereof according to any one of claims 1 to 6, **characterized in that** the antigen-binding fragment is a Fab, a Fab', a F(ab')2, a single chain variable fragment, or a disulphide-stabilised variable fragment.

8. The anti-GCC antibody or the antigen-binding fragment thereof according to any one of claims 1 to 7, **characterized by** comprising an amino acid sequence having at least 80% identity to any one of the sequences of SEQ ID NO: 43 to SEQ ID NO: 47, or comprising an amino acid sequence having at least 80% identity to SEQ ID NO: 57 and SEQ ID NO: 58;

preferably, the amino acid sequence of the anti-GCC antibody or the antigen-binding fragment thereof is set forth in any one of the sequences of SEQ ID NO: 43 to SEQ ID NO: 47.

9. An isolated nucleic acid, **characterized in that** the isolated nucleic acid encodes the anti-GCC antibody or the antigen-binding fragment thereof according to any one of claims 1 to 8.

10. A vector, **characterized by** comprising the isolated nucleic acid according to claim 9.

11. A host cell, **characterized by** comprising the isolated nucleic acid according to claim 9 or the vector according to claim 10.

12. A chimeric antigen receptor, **characterized by** comprising a polypeptide, the polypeptide comprising:

(1) an extracellular antigen-binding domain comprising the anti-GCC antibody or the antigen-binding fragment according to any one of claims 1 to 8;

(2) a transmembrane domain; and
(3) an intracellular signalling domain.

13. The chimeric antigen receptor according to claim 12, **characterized in that** the anti-GCC antibody or the antigen-binding fragment is a single chain variable fragment.

14. The chimeric antigen receptor according to any one of claims 12 to 13, **characterized by** further comprising a signal peptide located at the N-terminus of the polypeptide;

   preferably, the signal peptide is derived from a CD8α molecule, a CD28 molecule, or a CD4 molecule;
   preferably, the signal peptide comprises an amino acid sequence set forth in SEQ ID NO: 61, or an amino acid sequence having at least 85% identity to SEQ ID NO: 61.

15. The chimeric antigen receptor according to any one of claims 12 to 14, **characterized by** further comprising a hinge domain located between the C-terminus of the extracellular antigen-binding domain and the N-terminus of the transmembrane domain;

   preferably, the hinge region is derived from IgG4Fc CH2CH3, CD28, or CD8α;
   preferably, the hinge region comprises an amino acid sequence set forth in SEQ ID NO: 62, or an amino acid sequence having at least 85% identity to SEQ ID NO: 62.

16. The chimeric antigen receptor according to any one of claims 12 to 15, **characterized in that** the transmembrane domain is derived from one or more of transmembrane regions of CD8α, CD4, CD28, CD137, CD80, CD86, CD152, and PD1 molecules;
   preferably, the transmembrane domain comprises an amino acid sequence set forth in SEQ ID NO: 63, or an amino acid sequence having at least 85% identity to SEQ ID NO: 63.

17. The chimeric antigen receptor according to any one of claims 12 to 16, **characterized in that** the intracellular signalling domain comprises a costimulatory signalling domain;

   preferably, the costimulatory signalling domain is derived from the following costimulatory molecules: CD27, CD28, CD137, OX40, CD30, CD40, CD3, HVEM, ICOS, Myd88, LFA-1, ICOS, CD2, CD7, LIGHT, NKG2C, B7-H3, a ligand of CD83, and combinations thereof;
   preferably, the costimulatory signalling domain comprises a cytoplasmic domain of CD28 and/or a cytoplasmic domain of CD137;
   preferably, the costimulatory signalling domain comprises an amino acid sequence set forth in SEQ ID NO: 64, or an amino acid sequence having at least 85% identity to SEQ ID NO: 64.

18. The chimeric antigen receptor according to any one of claims 12 to 17, **characterized in that** the intracellular signalling domain comprises a primary intracellular signalling domain;

   preferably, the primary intracellular signalling domain is derived from CD3ζ;
   preferably, the primary intracellular signalling domain comprises an amino acid sequence set forth in SEQ ID NO: 65, or an amino acid sequence having at least 85% identity to SEQ ID NO: 65.

19. The chimeric antigen receptor according to any one of claims 12 to 18, **characterized in that** the chimeric antigen receptor has an amino acid sequence set forth in any one of SEQ ID NO: 49 to SEQ ID NO: 53 or in any one of SEQ ID NO: 71 to SEQ ID NO: 75.

20. An isolated nucleic acid, **characterized in that** the isolated nucleic acid encodes the chimeric antigen receptor according to any one of claims 12 to 19.

21. A vector, **characterized by** comprising the isolated nucleic acid according to claim 20.

22. A cell, **characterized by** comprising the chimeric antigen receptor according to any one of claims 12 to 19, comprising the isolated nucleic acid according to claim 20, or comprising the vector according to claim 21;
   preferably, the cell is an immune cell.

23. The cell according to claim 22, **characterized in that** the cell is selected from: a T cell, a NK cell, a peripheral blood mononuclear cell, a hematopoietic stem cell, a pluripotent stem cell, an embryonic stem cell, and combinations thereof.

24. A pharmaceutical composition, **characterized by** comprising the anti-GCC antibody or the antigen-binding fragment thereof according to any one of claims 1 to 8, or comprising the chimeric antigen receptor according to any one of claims 12 to 19, or comprising the cell according to claim 22 or 23;
preferably, the pharmaceutical composition further comprises one or more pharmaceutically acceptable excipients.

25. Use of the anti-GCC antibody or the antigen-binding fragment thereof according to any one of claims 1 to 8 or the chimeric antigen receptor according to any one of claims 12 to 19 in immunohistochemical detection or in the preparation of a detection kit.

26. Use of the anti-GCC antibody or the antigen-binding fragment thereof according to any one of claims 1 to 8, the chimeric antigen receptor according to any one of claims 12 to 19, or the cell according to claim 22 or 23 in the preparation of a medicament for treating or preventing a GCC-related disease or condition;

   preferably, the GCC-related disease or condition is a tumour;
   preferably, the GCC-related disease or condition is a gastrointestinal tract tumour;
   preferably, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

27. The anti-GCC antibody or the antigen-binding fragment thereof according to any one of claims 1 to 8, the chimeric antigen receptor according to any one of claims 12 to 19, or the cell according to claim 22 or 23, for use in treating or preventing a GCC-related disease or condition;

   preferably, the GCC-related disease or condition is a tumour;
   preferably, the GCC-related disease or condition is a gastrointestinal tract tumour;
   preferably, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

28. A method for treating or preventing a GCC-related disease or condition, **characterized by** comprising the step of administering to a subject in need thereof an effective amount of the anti-GCC antibody or the antigen-binding fragment thereof according to any one of claims 1 to 8, the chimeric antigen receptor according to any one of claims 12 to 19, or the cell according to claim 22 or 23;

   preferably, the GCC-related disease or condition is a tumour;
   preferably, the GCC-related disease or condition is a gastrointestinal tract tumour;
   preferably, the GCC-related disease or condition is one or more selected from gastrointestinal cancer, colorectal cancer, gastric cancer, oesophageal cancer, esophagogastric junction cancer, small intestine cancer, pancreatic cancer, and liver cancer.

**Human GCC-his protein**

FIG. 1A

**Mouse GCC-his protein**

FIG. 1B

**293T-human GCC.Luc**

- ■ 22G5-Rabbit IgG
- ● PF0098-Rabbit IgG
- + Rabbit IgG

*FIG. 2A*

**293T-mouse GCC.Luc**

- ■ 22G5-Rabbit IgG
- ● PF0098-Rabbit IgG
- + Rabbit IgG

*FIG. 2B*

*FIG. 2C*

*FIG. 3*

*FIG. 4A*

*FIG. 4B*

**CT26-mouse GCC.Luc (E/T=1.25:1)**

FIG. 4C

**LS1034.Luc (E/T=5:1)**

*FIG. 5A*

**LS1034.Luc (E/T=2.5:1)**

*FIG. 5B*

FIG. 5C

293T.Luc (E/T=5:1)

FIG. 6A

293T.Luc (E/T=2.5:1)

FIG. 6B

293T.Luc (E/T=1.25:1)

FIG. 6C

**CT26-mouse GCC.luc (E/T=2.5:1)**

FIG. 7A

**LS1034.luc (E/T=2.5:1)**

FIG. 7B

**293T.luc (E/T=2.5:1)**

*FIG. 7C*

**Baseline release from cells**

*FIG. 7D*

# INTERNATIONAL SEARCH REPORT

| International application No. |
|---|
| **PCT/CN2024/120038** |

| A. | CLASSIFICATION OF SUBJECT MATTER |
|---|---|

C07K16/40(2006.01)i; C07K19/00(2006.01)i; C12N15/13(2006.01)i; C12N15/62(2006.01)i; C12N15/85(2006.01)i; C12N5/078(2010.01)i; A61K39/395(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC: C07K16/-; C07K19/-; C12N15/-; C12N5/-; A61K39/-; A61P35/-; C07K14/-; G01N33/-

Documentation searched other than minimum documentation to the extent that such documents are included in the fields searched

Electronic data base consulted during the international search (name of data base and, where practicable, search terms used)

CNTXT, ENTXTC, WPABSC, DWPI, CJFD, CNKI, ISI WEB OF SCIENCE: 单抗, 抗体, 抗原结合, 鸟苷酸环化酶C, 嵌合抗原受体, antibody, antibodies, Ab, CAR, GCC, GUCY2C, guanylyl cyclase C, GAPDYSSNAFNL. GWSFCKS, DHGYYDV, GGGSSYYTGELYFNL, GNTYLDL; 中国专利生物序列检索系统, GENBANK+EMBL+DDBJ, LENS: 对SEQ ID NO: 11-15, 31-35的序列检索, China Patent Biological Sequence Search System, GENBANK+EMBL+DDBJ, LENS: search for SEQ ID NOs: 11-15 and 31-35

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2023125349 A1 (SHANDONG SIMCERE BIOPHARMACEUTICAL CO., LTD.) 06 July 2023 (2023-07-06) claims 1-24 | 1-27 |
| A | CN 104395470 B (MILLENNIUM PHARMACEUTICALS, INC.) 06 February 2018 (2018-02-06) claims 1-9 and 14-27 | 1-27 |
| A | CN 116535514 A (INNOVATIVE CELLULAR THERAPEUTICS CO., LTD.) 04 August 2023 (2023-08-04) claims 1-10 | 1-27 |
| A | CN 116601167 A (TAKEDA PHARMACEUTICAL CO., LTD.) 15 August 2023 (2023-08-15) claims 1-41 | 1-27 |

✓ Further documents are listed in the continuation of Box C.  ✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 December 2024** | **26 December 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/120038**

### C. DOCUMENTS CONSIDERED TO BE RELEVANT

| Category* | Citation of document, with indication, where appropriate, of the relevant passages | Relevant to claim No. |
|---|---|---|
| A | US 2023146072 A1 (PFIZER) 11 May 2023 (2023-05-11)<br>claims 1-19 | 1-27 |
| A | 姚镇东等 (YAO, Zhendong et al.). "鸟苷酸环化酶C在结直肠癌诊治中的研究进展 (Progress in Research on Guanylyl Cyclase C in Diagnosis and Treatment of Colorectal Cancer)"<br>*胃肠病学 (Chinese Journal of Gastroenterology),*<br>Vol. 21, No. (10), 25 October 2016 (2016-10-25), 626-628<br>ISSN: 1008-7125,<br>　　page 627, left-hand column, last paragraph to right-hand column, paragraph 1 | 1-27 |
| A | Michael S. Magee et al. "GUCY2C-Directed CAR-T Cells Oppose Colorectal Cancer Metastases without Autoimmunity"<br>*Oncoimmunology,* Vol. 5, No. (10), 14 October 2016 (2016-10-14), e1227897<br>ISSN: 2162-402X,<br>　　abstract, and figure 1 | 1-27 |

Form PCT/ISA/210 (second sheet) (July 2022)

**INTERNATIONAL SEARCH REPORT**

International application No.

**PCT/CN2024/120038**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

 a.  ☑ forming part of the international application as filed.

 b.  ☐ furnished subsequent to the international filing date for the purposes of international search (Rule 13*ter*.1(a)),

  ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
|---|
| **PCT/CN2024/120038** |

**Box No. II        Observations where certain claims were found unsearchable (Continuation of item 2 of first sheet)**

This international search report has not been established in respect of certain claims under Article 17(2)(a) for the following reasons:

1. ☑ Claims Nos.: **28**
   because they relate to subject matter not required to be searched by this Authority, namely:

   The technical solution of claim 28 relates to a method for treatment of a living human or animal body, and therefore said claim does not comply with PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

Claims 1-27 relate to five different anti-GCC antibodies in the present application, which antibodies differ in their respective light and heavy chain variable regions and CDR sequences, that is, there is no same structure among the antibodies. Therefore, the five inventions involving different antibodies do not have a same or corresponding technical feature, and obviously do not have a special technical feature that defines a contribution which the inventions make over the prior art, do not have a technical relationship therebetween, do not fall within a single general inventive concept, and therefore do not comply with the requirement of unity of invention and do not comply with PCT Rule 13.1.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

| International application No. |
|---|
| **PCT/CN2024/120038** |

| Patent document cited in search report | | | Publication date (day/month/year) | Patent family member(s) | | | Publication date (day/month/year) |
|---|---|---|---|---|---|---|---|
| WO | 2023125349 | A1 | 06 July 2023 | None | | | |
| CN | 104395470 | B | 06 February 2018 | GEP | 201706737 | B | 25 September 2017 |
| | | | | MA | 37569 | B1 | 31 August 2020 |
| | | | | HK | 1208049 | A1 | 19 February 2016 |
| | | | | MY | 188442 | A | 09 December 2021 |
| | | | | ZA | 201407723 | B | 30 January 2019 |
| | | | | US | 9273146 | B1 | 01 March 2016 |
| | | | | CY | 1122557 | T1 | 27 January 2021 |
| | | | | JP | 2015516985 | A | 18 June 2015 |
| | | | | JP | 6472746 | B2 | 20 February 2019 |
| | | | | HUE | 046404 | T2 | 28 February 2020 |
| | | | | EA | 201491977 | A1 | 30 April 2015 |
| | | | | EA | 034689 | B1 | 06 March 2020 |
| | | | | UA | 117910 | C2 | 25 October 2018 |
| | | | | RS | 59370 | B1 | 29 November 2019 |
| | | | | CR | 20140497 | A | 10 February 2015 |
| | | | | LT | 2841575 | T | 10 October 2019 |
| | | | | DK | 2841575 | T3 | 23 September 2019 |
| | | | | PL | 2841575 | T3 | 31 January 2020 |
| | | | | JP | 2017131245 | A | 03 August 2017 |
| | | | | JP | 6517267 | B2 | 22 May 2019 |
| | | | | SI | 2841575 | T1 | 29 November 2019 |
| | | | | CA | 2871614 | A1 | 31 October 2013 |
| | | | | CA | 2871614 | C | 31 August 2021 |
| | | | | TN | 2014000454 | A1 | 30 March 2016 |
| | | | | US | 2013287783 | A1 | 31 October 2013 |
| | | | | US | 9000129 | B2 | 07 April 2015 |
| | | | | IL | 235307 | A0 | 31 December 2014 |
| | | | | IL | 235307 | B | 31 July 2019 |
| | | | | AR | 090884 | A1 | 10 December 2014 |
| | | | | SA | 113340502 | B1 | 17 September 2015 |
| | | | | NZ | 701601 | A | 30 September 2016 |
| | | | | PE | 20142322 | A1 | 25 January 2015 |
| | | | | CO | 7280144 | A2 | 29 May 2015 |
| | | | | AU | 2013251312 | A1 | 30 October 2014 |
| | | | | AU | 2013251312 | B2 | 22 March 2018 |
| | | | | DOP | 2014000242 | A | 30 November 2014 |
| | | | | US | 2016130344 | A1 | 12 May 2016 |
| | | | | PH | 12014502399 | A1 | 22 December 2014 |
| | | | | HRP | 20191690 | T1 | 27 December 2019 |
| | | | | ECSP | 14028523 | A | 30 September 2015 |
| | | | | ME | 03560 | B | 20 July 2020 |
| | | | | BR | 112014026742 | A2 | 11 July 2017 |
| | | | | WO | 2013163633 | A1 | 31 October 2013 |
| | | | | KR | 20150004882 | A | 13 January 2015 |
| | | | | KR | 102046435 | B1 | 19 November 2019 |
| | | | | MX | 2014013081 | A | 12 January 2015 |
| | | | | MX | 362020 | B | 04 January 2019 |
| | | | | PT | 2841575 | T | 11 October 2019 |
| | | | | CL | 2014002911 | A1 | 30 January 2015 |

Form PCT/ISA/210 (patent family annex) (July 2022)

## INTERNATIONAL SEARCH REPORT
### Information on patent family members

| International application No. |
| --- |
| **PCT/CN2024/120038** |

| Patent document cited in search report | | | Publication date (day/month/year) | Patent family member(s) | | | Publication date (day/month/year) |
| --- | --- | --- | --- | --- | --- | --- | --- |
| | | | | SG | 11201406855 | TA | 27 November 2014 |
| | | | | EP | 2841575 | A1 | 04 March 2015 |
| | | | | EP | 2841575 | A4 | 15 June 2016 |
| | | | | EP | 2841575 | B1 | 26 June 2019 |
| | | | | ES | 2749181 | T3 | 19 March 2020 |
| | | | | TW | 201348258 | A | 01 December 2013 |
| | | | | TWI | 631137 | B | 01 August 2018 |
| CN | 116535514 | A | 04 August 2023 | None | | | |
| CN | 116601167 | A | 15 August 2023 | CO | 2023009102 | A2 | 21 July 2023 |
| | | | | EP | 4259656 | A1 | 18 October 2023 |
| | | | | PE | 20240049 | A1 | 09 January 2024 |
| | | | | MX | 2023006774 | A | 12 July 2023 |
| | | | | JP | 2023553159 | A | 20 December 2023 |
| | | | | AU | 2021397881 | A1 | 27 July 2023 |
| | | | | AU | 2021397881 | A9 | 19 September 2024 |
| | | | | TW | 202237638 | A | 01 October 2022 |
| | | | | WO | 2022123316 | A1 | 16 June 2022 |
| | | | | CL | 2023001666 | A1 | 19 January 2024 |
| | | | | CA | 3201582 | A1 | 16 June 2022 |
| | | | | AR | 124288 | A1 | 15 March 2023 |
| | | | | IL | 303535 | A | 01 August 2023 |
| | | | | KR | 20230118913 | A | 14 August 2023 |
| | | | | US | 2024050473 | A1 | 15 February 2024 |
| | | | | ECSP | 23050855 | A | 31 August 2023 |
| US | 2023146072 | A1 | 11 May 2023 | WO | 2021205325 | A1 | 14 October 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023120440 W
- US 4816567 A
- WO 2019224716 A2

### Non-patent literature cited in the description

- **KABAT et al.** Sequences of Proteins of Immunological Interest. HealthService, National Institutes of Health, 1991
- **MARTIN et al.** *ProcNatl Acad Sci (USA)*, 1989, vol. 86, 9268-9272
- AbMTM, A Computer Program for ModelingVariable Regions of Antibodies. Oxford Molecular, Ltd
- **LEFRANC et al.** *Dev. Comparat. Immunol.*, 2003, vol. 27, 55-77
- **CHOTHIA et al.** *J. Mol. Biol.*, 1986, vol. 196, 901-17
- **CHOTHIA et al.** *Nature*, 1989, vol. 342, 877-83
- **MACCALLUM et al.** *J. Mol. Biol.*, 1996, vol. 5, 732-745
- **KÖHLER G** ; **MILSTEIN C.** Continuous cultures of fused cells secreting antibody of predefined specificity [J. *nature*, 1975, vol. 256 (5517), 495
- **JONES et al.** *Nature*, 1986, vol. 321, 522, 525
- **REICHMANN et al.** *Nature*, 1988, vol. 332, 323, 329
- **PRESTA**. *Curr. Op. Struct. Biol.*, 1992, vol. 2, 593-596
- **CLARK**. *Immunol. Today*, 2000, vol. 21, 397, 402
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunology, Immunotherapy*, 2001, vol. 50 (1), 51-59
- Remington's Pharmaceutical Sciences. Mack Publishing Company, 1995